# EUROPEAN PATENT SPECIFICATION

(11) **EP 4 534 011 B1**
(45) Date of publication and mention of the grant of the patent: **23.09.2026**
(21) Application number: 23815739.0
(22) Date of filing: 15.05.2023
(51) Int. Cl.: A61B 5/16, A61B 5/38, G06N 20/00

(54) **MOOD ESTIMATING PROGRAM**
STIMMUNGSSCHÄTZUNGSPROGRAMM
PROGRAMME D'ESTIMATION D'HUMEUR

(30) Priority: 30.05.2022 JP 2022087801
(43) Date of publication of application: 09.04.2025
(73) Proprietor: NATIONAL INSTITUTE OF INFORMATION AND COMMUNICATIONS TECHNOLOGY, Tokyo 184-8795 (JP)
(72) Inventor: WATANABE, Hiroki, Koganei-shi, Tokyo 184-8795 (JP); IHARA, Aya, Koganei-shi, Tokyo 184-8795 (JP); NARUSE, Yasushi, Koganei-shi, Tokyo 184-8795 (JP); FUSEDA, Kohei, Kawagoe-shi, Saitama 350-1121 (JP)
(74) Representative: Mewburn Ellis LLP
(86) International application number: PCT/JP2023/018025
(87) International publication number: WO 2023/233979

(56) References cited:
- WO-A1-2021/241138
- WO-A1-2022/102721
- CN-A- 109 157 231
- JP-A- 2018 082 730
- JP-A- 2020 091 591
- US-A1- 2019 246 936
- KANG MIN ET AL: "The deep learning method for predict beck's depression inventory score using EEG", 2021 INTERNATIONAL CONFERENCE ON INFORMATION AND COMMUNICATION TECHNOLOGY CONVERGENCE (ICTC), IEEE, 20 October 2021 (2021-10-20), pages 490 - 493, XP034038956, DOI: 10.1109/ICTC52510.2021.9620922

## Description

### Technical Field

The present invention relates to a mood estimation program.

### Background Art

So far, there has been reported a study that discriminates between major depressive disorder and healthy person, and discriminates between a person with high mood of depression and a person with low mood of depression in a non-clinical group, by machine learning and deep learning using features of electroencephalograms. For example, NPL 1 below discloses a method of measuring an electroencephalogram when a subject is performing a task for auditory stimulation, and identifying whether or not the subject is suffering from major depressive disorder, using, as features, latencies, amplitudes, and the like of event-related potential components (N1, P300).

### Citation List

### Non Patent Literature

NPL 1: Jang, K. et al. (2021). "Machine learning-based electroencephalographic phenotypes of Schizophrenia and Major Depressive Disorder". Frontiers in Psychiatry, 12:745458 (Published online version on October 13, 2021, doi: 10.3389/fpsyt.2021.745458). CN109157231A and US2019/246936A1 disclose exemplary eeg analysis devices and methods.

### Summary of Invention

### Technical Problem

In recent years, with the development of media, especially the spread of the Internet, information received by people has explosively increased, but the capacity of the brain of modern people has not correspondingly increased. Stress, mental disorder, and deterioration in productivity due to excessive information have become social problems. In addition, not only the amount of information but also the content of information affects mental health. During the spread of the new coronavirus, the number of people suffering from mental illness has increased. Recent studies have reported that people exposed more to negative pandemic-related news suffer from mental illness.

There is a large individual difference in how to perceive negative information. For example, it has been reported that there is attention bias that a person with depression is more likely to pay attention to negative information than a person without depression. In addition, it has been reported that cognitive processing is affected in a depressed person in order to allocate resources to processing of emotional information.

However, in the above-described conventional technology, electroencephalograms are measured in a special state of performing a task for a simple sound stimulus (a sound stimulus delivered at a fixed stimulus interval of 2000 milliseconds in 85 [decibels]), and electroencephalograms for sound information that is daily heard are not used for estimating mood.

The present invention has been made in view of such circumstances, and an object thereof is to provide an estimation device that estimates a mental state of an individual, particularly a level of depressed mood, based on a brain response to daily voice information, or the like.

### Solution to Problem

In order to solve the above-described problem, the present invention adopts the configuration as defined in the claims.

A mood estimation program according to a first aspect causes a computer to perform: a target person electroencephalogram acquisition step of acquiring a target person electroencephalogram which is an electroencephalogram of a target person when listening to a voice uttering a sentence; an electroencephalogram encoding step of generating an electroencephalogram feature from an electroencephalogram of a person when listening to a voice uttering a sentence, the electroencephalogram encoding step generating a target person electroencephalogram feature as the electroencephalogram feature from the target person electroencephalogram; and an estimation step of estimating a target person mood score which is a mood score indicating a level of depressed mood of the target person by inputting the target person electroencephalogram feature to an estimation model, the estimation model receiving at least the electroencephalogram feature as an input and estimating a mood score indicating a level of depressed mood of the person, the estimation model being generated by performing machine learning using a plurality of training data sets, each of the plurality of training data sets being formed by associating a subject mood score which is the mood score indicating a level of depressed mood of a learning subject with at least a subject electroencephalogram feature which is the electroencephalogram feature generated from an electroencephalogram of the learning subject when listening to a voice uttering a sentence, performing the machine learning including a training step of training the estimation model so that the mood score estimated by the estimation model when the subject electroencephalogram feature is received as an input matches the subject mood score for each of the plurality of training data sets.

In this configuration, the mood estimation program causes a computer to estimate the target person mood score indicating a level of depressed mood of the target person based on the target person electroencephalogram feature generated from an electroencephalogram of the target person when the target person is listening to "a voice uttering a sentence" such as a news voice or a conversation voice. The mood estimation program causes a computer to estimate the target person mood score by inputting the target person electroencephalogram feature to the estimation model generated by performing the machine learning using the plurality of training data sets.

The training data set is configured so that at least an electroencephalogram feature (the subject electroencephalogram feature) generated from an electroencephalogram of a person (the learning subject) when listening to a voice uttering a sentence is associated with a mood score (the subject mood score) indicating a level of depressed mood of the person. In addition, performing the machine learning includes a training step of training the estimation model so that the mood score estimated by the estimation model from the subject electroencephalogram feature matches the subject mood score for each of the plurality of training data sets.

The present inventors verified the estimation accuracy for the estimation model generated by performing the machine learning using the plurality of training data sets configured by associating the subject mood score with the subject electroencephalogram feature, and obtained the following verification results. That is, it was confirmed that the AUC (Area Under the Roc Curve) of the estimation model was "0.73". In addition, the estimation model identified 66% of people with high level of depressed mood (people whose BDI score calculated from answers to the Beck Depression Inventory (BDI) is 14 or more) as having high level of depressed mood.

Therefore, the computer can estimate the target person mood score with high accuracy by inputting, to the estimation model, the target person electroencephalogram feature generated from the electroencephalogram of the target person when listening to "a voice uttering a sentence" such as a news voice or a conversation voice.

In particular, the "voice uttering a sentence" such as a news voice or a conversation voice is not an unusual (special) sound described in NPL 1, but is a voice that the target person ordinarily hears, that is, daily voice information. Therefore, the computer can estimate the target person mood score indicating the level of the depressed mood of the target person from the electroencephalogram (the brain response) of the target person with respect to the daily voice information.

A mood estimation program according to a second aspect may cause, in the mood estimation program according to the first aspect, the computer to perform in the electroencephalogram encoding step, generating an electroencephalogram feature corresponding to a category into which the sentence is classified, as the electroencephalogram feature, based on an electroencephalogram of a person when listening to a voice uttering a sentence classified into any of at least three categories of negative, neutral, and positive, and information indicating into which of the at least three categories the sentence is classified, the estimation model estimating the mood score when an electroencephalogram feature corresponding to a category into which the sentence is classified is input as the electroencephalogram feature, the subject electroencephalogram feature including a subject first electroencephalogram feature which is an electroencephalogram feature corresponding to the category of negative generated from an average of a plurality of electroencephalograms each of which is an electroencephalogram of the learning subject when listening to a voice uttering a sentence classified into the category of negative, a subject second electroencephalogram feature which is an electroencephalogram feature corresponding to the category of neutral generated from an average of a plurality of electroencephalograms each of which is an electroencephalogram of the learning subject when listening to a voice uttering a sentence classified into the category of neutral, and a subject third electroencephalogram feature which is an electroencephalogram feature corresponding to the category of positive generated from an average of a plurality of electroencephalograms each of which is an electroencephalogram of the learning subject when listening to a voice uttering a sentence classified into the category of positive, performing the machine learning including, for each of the plurality of training data sets, a first training step of training the estimation model so that the mood score estimated by the estimation model when the subject first electroencephalogram feature is input as the electroencephalogram feature corresponding to the category of negative matches the subject mood score, a second training step of training the estimation model so that the mood score estimated by the estimation model when the subject second electroencephalogram feature is input as the electroencephalogram feature corresponding to the category of neutral matches the subject mood score, and a third training step of training the estimation model so that the mood score estimated by the estimation model when the subject third electroencephalogram feature is input as the electroencephalogram feature corresponding to the category of positive matches the subject mood score, and the program may cause the computer to further perform a classification information acquisition step of acquiring classification information indicating into which of the at least three categories a sentence the target person is listening to as a voice is classified, and in the estimation step, estimating the target person mood score by inputting to the estimation model, as an electroencephalogram feature corresponding to a category indicated by the classification information, the target person electroencephalogram feature corresponding to a category indicated by the classification information generated, in the electroencephalogram encoding step, based on the target person electroencephalogram of the target person when listening to a voice uttering a sentence classified into a category indicated by the classification information and the classification information.

In this configuration, the training data set is configured by associating the subject mood score with an electroencephalogram feature (the first electroencephalogram feature of the subject, the second electroencephalogram feature of the subject, and the third electroencephalogram feature of the subject) corresponding to each of at least three categories of negative, neutral, and positive. The estimation model is generated by performing the machine learning including the first training step, the second training step, and the third training step using the training data set. Therefore, the estimation model can estimate the mood score indicating the level of depressed mood of the person based on the electroencephalogram feature corresponding to each of the categories.

The computer further executes a classification information acquisition step of acquiring the classification information. The classification information may be acquired from the outside of the computer. The computer may generate the classification information, and acquire the generated classification information in the classification information acquisition step. The classification information may be generated in an analog manner (for example, by a person classifying the sentence into any one of the at least three categories). In addition, the classification information may be generated by classifying the sentence into any of the at least three categories using a classification model or the like generated by performing machine learning using a plurality of training data sets each configured by a combination of a sentence and a classification result. The classification information may be generated on a rule basis or may be generated on a model basis. The computer generates the target person electroencephalogram feature corresponding to the category indicated by the classification information from the target person electroencephalogram of the target person when listening to a voice uttering a sentence classified into the category indicated by the classification information. Then, the computer estimates the target person mood score by inputting the target person electroencephalogram feature corresponding to the category indicated by the classification information to the estimation model.

Therefore, the computer can estimate the target person mood score indicating the level of the depressed mood of the target person based on the electroencephalogram of the target person when listening to the voice uttering the sentence and the classification information indicating whether the sentence is classified into at least one of the three categories of negative, neutral, and positive.

A mood estimation program according to a third aspect may cause, in the mood estimation program according to the first or second aspect, the computer to perform in the electroencephalogram encoding step, generating, as the electroencephalogram feature, at least one of a peak latency and an average amplitude before and after a peak of a predetermined component in an electroencephalogram response to a word of a person based on an electroencephalogram of the person when listening to a voice uttering a sentence and a start point of each word included in the sentence that the person is listening to as a voice, the subject electroencephalogram feature being at least one of a peak latency and an average amplitude before and after a peak of the predetermined component in an electroencephalogram response to the word of the learning subject generated based on an electroencephalogram of the learning subject when listening to a voice uttering a sentence and a start point of each word included in the sentence that the learning subject is listening to as a voice, the program may cause the computer to further perform an onset information acquisition step of acquiring onset information indicating a start point of each word included in a sentence that the target person is listening to as a voice, and in the estimation step, estimating the target person mood score by inputting to the estimation model, as the target person electroencephalogram feature, at least one of a peak latency and an average amplitude before and after a peak of the predetermined component in an electroencephalogram response to the word of the target person generated, in the electroencephalogram encoding step, based on the target person electroencephalogram and a start point of each word included in a sentence that the target person is listening to as a voice indicated by the onset information.

In this configuration, the electroencephalogram feature is at least one of a peak latency and an average amplitude before and after a peak of the predetermined component in an electroencephalogram response to a word included in a sentence. The predetermined component is, for example, at least one of components having peaks around 100 milliseconds, around 200 milliseconds, and around 400 milliseconds after word presentation. The training data set is configured by associating the subject mood score with at least one of a peak latency and an average amplitude before and after a peak of the predetermined component in an electroencephalogram response to a word of the learning subject. Then, the estimation model is generated by performing the machine learning using the training data set. Therefore, the estimation model can estimate the mood score indicating the level of depressed mood of the person based on at least one of the peak latency and the average amplitude before and after the peak of the predetermined component in the electroencephalogram response to the word included in the sentence.

The computer further executes an onset information acquisition step of obtaining the onset information. The computer generates, as the target person electroencephalogram feature, at least one of a peak latency and an average amplitude before and after a peak of the predetermined component in an electroencephalogram response to a word of the target person from the target person electroencephalogram and the onset information. Then, the computer estimates the target person mood score by inputting at least one of a peak latency and an average amplitude before and after a peak of the predetermined component in the electroencephalogram response to the word of the target person to the estimation model.

Therefore, the computer can estimate the target person mood score indicating the level of the depressed mood of the target person based on the electroencephalogram of the target person when listening to the voice uttering the sentence and the onset information indicating the start point of each word included in the sentence.

A mood estimation program according to a fourth aspect may cause, in the mood estimation program according to any one of the first to third aspects, the computer to perform in the electroencephalogram encoding step, generating, as the electroencephalogram feature, at least one of a peak latency and an average amplitude before and after a peak of a predetermined component in an electroencephalogram response following a voice envelope of a person based on an electroencephalogram of the person when listening to a voice uttering a sentence and the voice envelope of the voice that the person is listening to, the subject electroencephalogram feature being at least one of a peak latency and an average amplitude before and after a peak of the predetermined component in an electroencephalogram response following the voice envelope of the learning subject generated based on an electroencephalogram of the learning subject when listening to a voice uttering a sentence and a voice envelope of the voice that the learning subject was listening to, the program may cause the computer to further perform: an envelope information acquisition step of acquiring envelope information indicating a voice envelope of a voice the target person is listening to; and in the estimation step, estimating the target person mood score by inputting to the estimation model, as the target person electroencephalogram feature, at least one of a peak latency and an average amplitude before and after a peak of the predetermined component in an electroencephalogram response following the voice envelope of the target person generated, in the electroencephalogram encoding step, based on the target person electroencephalogram and a voice envelope of a voice the target person is listening to indicated by the envelope information.

In this configuration, the electroencephalogram feature is at least one of a peak latency and an average amplitude before and after a peak of the predetermined component in an electroencephalogram response following a voice envelope of a listening voice. The predetermined component is, for example, at least one of components having peaks around 50 milliseconds, around 150 milliseconds, and around 250 milliseconds in an electroencephalogram response analyzed based on a voice envelope. The training data set is configured by associating the subject mood score with at least one of a peak latency and an average amplitude before and after a peak of the predetermined component in an electroencephalogram response following a voice envelope of the learning subject. Then, the estimation model is generated by performing the machine learning using the training data set. Therefore, the estimation model can estimate the mood score indicating the level of depressed mood of the person based on at least one of the peak latency and the average amplitude before and after the peak of the predetermined component in the electroencephalogram response following the voice envelope.

Further, the computer performs an envelope information acquisition step of acquiring the envelope information. The computer generates, as the target person electroencephalogram feature, at least one of a peak latency of the predetermined component and an average amplitude before and after a peak in an electroencephalogram response following the voice envelope of the target person from the target person electroencephalogram and the envelope information. Then, the computer estimates the target person mood score by inputting at least one of a peak latency and an average amplitude before and after a peak of the predetermined component in the electroencephalogram response following the voice envelope of the target person to the estimation model.

Therefore, the computer can estimate the target person mood score indicating the level of the depressed mood of the target person based on the electroencephalogram of the target person when listening to the voice uttering the sentence and the envelope information indicating the voice envelope of the voice.

A mood estimation program according to a fifth aspect may be characterized in that, in the mood estimation program according to any one of the first to fourth aspects, the estimation model further receives, as an input, a subjective score indicating subjective evaluation felt by a person for a sentence after listening to a voice uttering the sentence, in addition to the electroencephalogram feature, and estimates the mood score based on the electroencephalogram feature and the subjective score that are input, and each of the plurality of training data sets is formed by associating the subject mood score with the subject electroencephalogram feature and a subject subjective score which is the subjective score indicating subjective evaluation felt by the learning subject for the sentence after listening to a voice uttering the sentence, and performing the machine learning includes a training step of training the estimation model so that the mood score estimated by the estimation model when the subject electroencephalogram feature and the subject subjective score are input matches the subject mood score for each of the plurality of training data sets, the program may cause a computer to further perform a target person subjective score acquisition step of acquiring a target person subjective score which is the subjective score indicating subjective evaluation that the target person felt for the sentence after listening to a voice uttering the sentence, and in the estimation step, estimating the target person mood score by inputting to the estimation model the target person electroencephalogram feature and the target person subjective score.

In this configuration, the training data set is formed so that the subject mood score is associated with the subject electroencephalogram feature and the subjective score (the subject subjective score) indicating the subjective evaluation felt by the learning subject for the sentence listened as a voice. In addition, performing the machine learning includes a training step of training the estimation model so that the mood score estimated by the estimation model when the subject electroencephalogram feature and the subject subjective score are input matches the subject mood score for each of the plurality of training data sets. Therefore, the estimation model can estimate the mood score indicating the level of depressed mood of the person based on the electroencephalogram feature and the subjective score indicating the subjective evaluation felt by the person with respect to the sentence listened as a voice.

The present inventors verified the estimation accuracy for the estimation model generated by performing the machine learning using the plurality of training data sets configured by associating the subject mood score with the subject electroencephalogram feature and the subject subjective score, respectively, and obtained the following verification results. That is, it was confirmed that the AUC of the estimation model was "0.83". In addition, the estimation model identified 78% of people with high level of depressive mood as having high level of depressive mood.

Furthermore, the computer executes a target person subjective score acquisition step of acquiring the target person subjective score. The computer estimates the target person mood score by inputting the target person electroencephalogram and the target person subjective score to the estimation model.

Therefore, the computer can estimate the target person mood score indicating the level of the depressed mood of the target person with high accuracy based on the electroencephalograms of the target person when listening to the voice uttering the sentence and the target person subjective score indicating the subjective evaluation felt by the target person for the sentence.

A mood estimation program according to a sixth aspect may cause, in the mood estimation program according to any one of the first to fifth aspects, the computer to further perform, an output step of outputting, to the target person, information corresponding to the target person mood score estimated in the estimation step.

In this configuration, the computer outputs (for example, notifies) information corresponding to the target person mood score to the target person. The information corresponding to the target person mood score may be the target person mood score itself. Furthermore, the information corresponding to the target person mood score may be information indicating the level of depressed mood of the target person indicated by the target person mood score. Furthermore, the information corresponding to the target person mood score may be information including advice to the target person corresponding to the target person mood score. For example, the computer can cause the target person to be aware of a state of mind such as depression of his/her own mood by outputting the target person mood score to the target person. For example, the computer can output, to the target person, information including advice to the target person, corresponding to the target person mood score, thereby urging the target person to take an action for maintaining mental health, such as blocking information that puts a heavy mental burden.

In a case where the target person mood score indicates that the target person has a high level of depressed mood, the information corresponding to the target person mood score may be information for relaxing the target person, for example, music, video, or the like for relaxing the target person. The computer can output information according to the level of depressed mood of the target person indicated by the target person mood score to the target person as "information corresponding to the target person mood score".

In particular, the computer estimates the target person mood score from an electroencephalogram of the target person when the target person is listening to "a voice uttering a sentence" such as a news voice or a conversation voice, which the target person hears on a daily basis, instead of the unusual (special) sound described in NPL 1. Therefore, the computer estimates the target person mood score based on the electroencephalograms of the target person with respect to the daily speech information, and outputs the estimated target person mood score to the target person, thereby making the target person aware of his/her own state of mind, encouraging the target person to take action, and relaxing the target person.

Furthermore, as another aspect of the mood estimation program according to each of the above viewpoints, one aspect of the present invention may be a computer or other device that executes the mood estimation program according to each of the above viewpoints, or may be a storage medium that stores the mood estimation program according to each of the above viewpoints and is readable by the computer, other device, machine, or the like. Here, the computer-readable storage medium is a medium that accumulates information such as a program by electrical, magnetic, optical, mechanical, or chemical action.

### Advantageous Effects

According to the disclosure, it is possible to provide an estimation device or the like that estimates a mental state of an individual, particularly a level of depressed mood, based on a brain response to daily voice information.

### Brief Description of Drawings

[Fig. 1] Fig. 1 schematically shows an example of a scene to which the present disclosure is applied.
[Fig. 2] Fig. 2 schematically shows an example of a hardware configuration of a model generation device according to the embodiment.
[Fig. 3] Fig. 3 schematically shows an example of a hardware configuration of an estimation device according to the embodiment.
[Fig. 4] Fig. 4 schematically shows an example of a software configuration of the model generation device according to the embodiment.
[Fig. 5] Fig. 5 schematically shows an example of each process of feature generation processing and training processing executed by the model generation device according to the embodiment.
[Fig. 6] Fig. 6 schematically shows an example of a software configuration of the estimation device according to the embodiment.
[Fig. 7] Fig. 7 schematically shows an example of each process of the feature generation processing and the mood estimation processing executed by the estimation device according to the embodiment.
[Fig. 8] Fig. 8 shows an example of a processing procedure of the model generation device according to the embodiment.
[Fig. 9] Fig. 9 shows an example of a processing procedure of the estimation device according to the embodiment.

### Description of Embodiments

Hereinafter, an embodiment (hereinafter, also referred to as "present embodiment") according to one aspect of the present disclosure will be described with reference to the drawings. However, the present embodiment described below is merely an example of the present invention in all respects. It goes without saying that various improvements and modifications can be made without departing from the scope of the present invention. That is, in carrying out the present invention, a specific configuration according to the embodiment may be appropriately adopted. Note that data appearing in the present embodiment has been described in a natural language. More specifically, the data is specified in a pseudo language, a command, a parameter, a machine language, or the like that can be recognized by a computer.

### §1 Application example

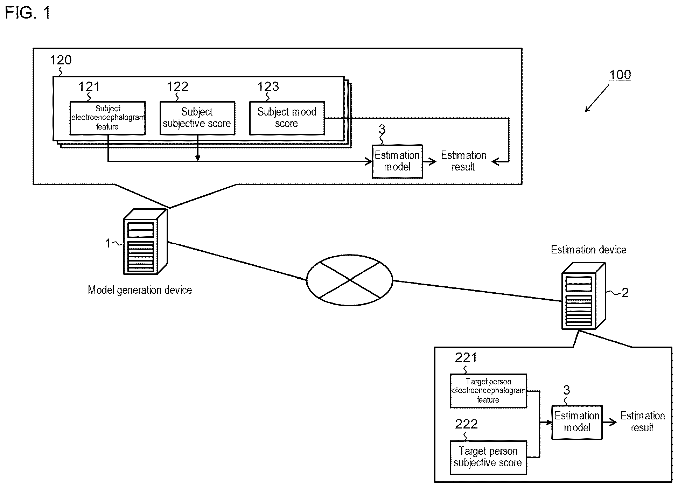

Fig. 1 schematically shows an example of a scene to which the present invention is applied. A mood estimation system 100 according to the present embodiment includes a model generation device 1 and an estimation device 2.

The model generation device 1 according to the present embodiment is a computer configured to perform machine learning of an estimation model 3. The model generation device 1 performs machine learning of the estimation model 3 using the plurality of training data sets 120.

The estimation model 3 is configured to execute an estimation task of estimating a mood score Sm indicating the level of depressed mood of a person (in other words, to output an output value corresponding to a result of executing the estimation task) when given at least an electroencephalogram feature Fw generated from an electroencephalogram of the person (learning subject and target person) when listening to a voice uttering a sentence. In the present embodiment, the estimation model 3 receives, in addition to the electroencephalogram feature Fw generated from the electroencephalogram of the person when listening to the voice uttering the sentence, a subjective score Ss indicating a subjective evaluation felt by the person for the sentence after listening, and estimates the mood score Sm from the electroencephalogram feature Fw and the subjective score Ss.

The "voice uttering a sentence" may be, for example, a voice uttering news (news voice) or a voice (conversation voice) in which another party of the conversation utters a conversation content (conversation sentence). The "voice uttering a sentence" may be a voice uttered by a person other than the "person whose electroencephalogram is to be measured", a machine, or the like, and is a voice (daily voice) that a person normally hears. In the present embodiment, a news voice is used as the "voice uttering a sentence".

In the present embodiment, a sentence (for example, news) that a person is listening to as a voice is classified into at least one of three categories of negative, neutral, and positive. A sentence that a person is listening to as a voice can generate at least one of negative, neutral, and positive emotions. In the following description, "negative", "neutral", and "positive" may be abbreviated as "Ng", "Nt", and "Ps", respectively.

The classification of sentences that a person listens to as a voice may be performed in an analog manner. For example, "categories in which a plurality of persons other than a person who listens to a voice uttering the sentences have classified the sentences" may be statistically processed to adopt as categories into which the sentences are classified. In addition, the sentence may be automatically or semiautomatically classified by a classification device or the like. For example, a sentence that a person listens to as a voice may be classified into one of the at least three categories described above using a classification model or the like generated by performing machine learning using a plurality of training data sets each including a combination of a sentence and a classification result (category). The sentence classification may be performed on a rule basis or a model basis.

The electroencephalogram may be an electroencephalogram of a person when the person when listening to a voice uttering a sentence. In the present embodiment, the electroencephalogram is specified from the electroencephalogram measured at each of a plurality of (for example, three) electroencephalogram measurement points for a person when the person is listening to a voice uttering a sentence. However, it is not essential that the electroencephalogram is specified from the electroencephalogram measured at each of the plurality of electroencephalogram measurement points. The electroencephalogram may be an electroencephalogram measured at one electroencephalogram measurement point for a person when the person is listening to a voice uttering a sentence.

Corresponding to the sentence that the person listens to as the voice being classified into at least three categories of "Ng", "Nt", and "Ps", the electroencephalogram feature Fw corresponding to each of the at least three categories is generated from the electroencephalogram of a person when the person is listening to the voice uttering the sentence. For example, a first electroencephalogram feature Fw(1) corresponding to the category "Ng" is generated from the electroencephalogram of the person when listening to the voice uttering the sentence classified into the category "Ng". Furthermore, a second electroencephalogram feature Fw(2) corresponding to the category "Nt" is generated from the electroencephalogram of the person when listening to the voice uttering the sentence classified into the category "Nt". Similarly, a third electroencephalogram feature Fw(3) corresponding to the category "Ps" is generated from the electroencephalogram of the person while listening to the voice uttering the sentence classified into the category "Ps". In a case where at least one of the first electroencephalogram feature Fw(1), the second electroencephalogram feature Fw(2), and the third electroencephalogram feature Fw(3) is given, the estimation model 3 estimates (outputs) the mood score Sm. In the following description, in a case where the first electroencephalogram feature Fw(1), the second electroencephalogram feature Fw(2), and the third electroencephalogram feature Fw(3) are not particularly distinguished, they may be collectively referred to as "electroencephalogram feature Fw".

The electroencephalogram feature Fw is at least one of a peak latency and an average amplitude before and after a peak of a predetermined component Pc of an electroencephalogram (electroencephalogram response) of a person when listening to a voice uttering a sentence. In the following description, "at least one of the peak latency of the predetermined component Pc and the average amplitude before and after the peak" may be referred to as "component feature Ifa". In the present embodiment, the "electroencephalogram of a person when listening to a voice uttering a sentence" is at least one of an "electroencephalogram response to a word (each word) included in a sentence that the person is listening to as a voice" and an "electroencephalogram response following a voice envelope of the voice". The "predetermined component Pc" may be, for example, at least one of components having peaks around 100 milliseconds, around 200 milliseconds, and around 400 milliseconds after word presentation. Furthermore, the "predetermined component Pc" may be, for example, at least one of components having peaks around 50 milliseconds (or around 100 milliseconds), around 150 milliseconds (or around 200 milliseconds), and around 250 milliseconds (or around 400 milliseconds) in the electroencephalogram response analyzed based on the voice envelope. Hereinafter, an example will be described in which the component feature Ifa of the "electroencephalogram response to a word included in a sentence that a person is listening to as a voice", that is, the component feature Ifa of the "electroencephalogram response to a word" of a person is used as the electroencephalogram feature Fw.

In the present embodiment, the subjective score Ss is information including five-grade evaluation for each of a difficulty level, an interest level, a valence, and a wakefulness level felt by a person for a sentence (voice) after listening to the voice uttering the sentence. The "difficulty level" indicates the difficulty level felt for the listened sentence (voice) by five-grade evaluation. The "interest level" indicates the interest level felt for the listened sentence (voice) by five-grade evaluation. The "valence" indicates whether the listened sentence (voice) is regarded as positive or negative by five-grade evaluation. The "wakefulness level" indicates the degree of emotional arousal (from "strongly aroused" to "weakly aroused") by five-grade evaluation. However, the subjective score Ss is not limited to the one indicating the five-grade evaluation for each of the difficulty level, the interest level, the valence, and the wakefulness level, and may be any one indicating the subjective evaluation felt by the person for the sentence after listening to the voice uttering the sentence.

In the present embodiment, the mood score Sm may be, for example, a BDI score calculated from answers to the Beck Depression Inventory (BDI) of a person. In addition, the mood score Sm may indicate whether or not the person depressed mood is high, and may indicate, for example, whether or not the BDI score is "14" or more. However, the mood score Sm is not limited to these examples, and may be any score as long as it can indicate the level of a person depressed mood.

Each training data set 120 used for the machine learning of the estimation model 3 includes a combination of the subject electroencephalogram feature 121, the subject subjective score 122, and the subject mood score 123. The subject electroencephalogram feature 121 is an electroencephalogram feature Fw generated from an electroencephalogram of the learning subject when listening to a voice uttering a sentence. The subject subjective score 122 is a subjective score Ss indicating the subjective evaluation felt by the learning subject for the sentence after listening to the voice uttering the sentence. The subject mood score 123 is a mood score Sm indicating the level of the depressed mood of the learning subject, and indicates the correct answer of the estimation task for the subject electroencephalogram feature 121 and the subject subjective score 122.

Performing machine learning in the present embodiment includes the following training step. That is, a training step of training the estimation model 3 so that a result (mood score Sm) of executing the estimation task of the estimation model 3 conforms to (matches) the subject mood score 123 when the subject electroencephalogram feature 121 and the subject subjective score 122 are given to the estimation model 3 for each training data set 120 is included. The model generation device 1 may train the estimation model 3 by, for example, machine learning by a support vector machine (linear support vector machine). The estimation model 3 generated by performing machine learning with the linear support vector machine can estimate whether a person depressed mood is high (for example, whether the BDI score is "14" or higher).

The estimation device 2 is an example of a computer that executes a mood estimation program (mood estimation program 82) according to the present invention, and is a computer configured to perform an estimation task using a trained machine learning model (estimation model 3) generated by the model generation device 1. In the present embodiment, the estimation device 2 uses the trained estimation model 3 to target person subjective score 9 (estimate a target person mood score 223 that is a mood score Sm indicating the level of depressed mood of the target person). The target person electroencephalogram feature 221 is an electroencephalogram feature Fw generated from the electroencephalogram of the target person when the target person is listening to the voice uttering the sentence. The target person subjective score 9 is a subjective score Ss indicating the subjective evaluation felt by the target person for the sentence after listening to the voice uttering the sentence.

Note that, in the present disclosure the subjective score Ss is not essential as an input to the estimation model 3. The estimation model 3 only needs to be able to receive at least an input of the electroencephalogram feature Fw and estimate the mood score Sm based on the input electroencephalogram feature Fw. Correspondingly, each training data set 120 may be configured by associating the subject mood score 123 with at least the subject electroencephalogram feature 121. In this case, the training step is only required to train the estimation model 3 so that the mood score Sm estimated by the estimation model 3 matches the subject mood score 123 when the subject electroencephalogram feature 121 is given to the estimation model 3 for each training data set 120. In a case where the estimation model 3 that estimates the mood score Sm only from the electroencephalogram feature Fw without requiring the subjective score Ss is used, the estimation device 2 can estimate the target person mood score 223 by giving only the target person electroencephalogram feature 221 to the estimation model 3.

In the example of Fig. 1, the model generation device 1 and the estimation device 2 are connected to each other via a network. The type of the network may be appropriately selected from, for example, the Internet, a wireless communication network, a mobile communication network, a telephone network, a dedicated network, and the like. However, the method of exchanging data between the model generation device 1 and the estimation device 2 is not limited to such an example, and may be appropriately selected according to the embodiment. For example, data may be exchanged between the model generation device 1 and the estimation device 2 using a storage medium.

Furthermore, in the example of Fig. 1, the model generation device 1 and the estimation device 2 are configured by separate computers. However, the configuration of the mood estimation system 100 according to the present embodiment is not limited to such an example, and may be appropriately determined according to the embodiment. For example, the model generation device 1 and the estimation device 2 may be an integrated computer. Furthermore, for example, at least one of the model generation device 1 and the estimation device 2 may be configured by a plurality of computers.

### §2 Configuration example

### [Hardware configuration]

### <Model generation device>

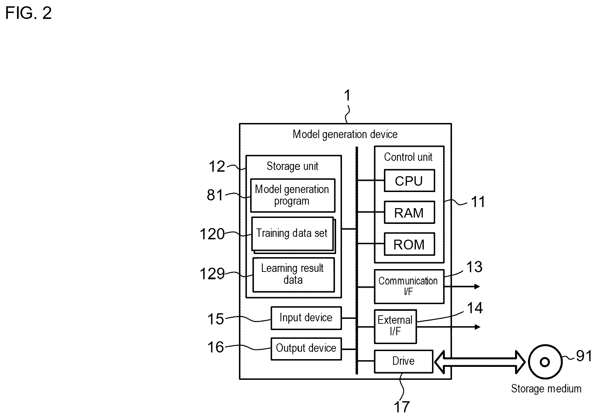

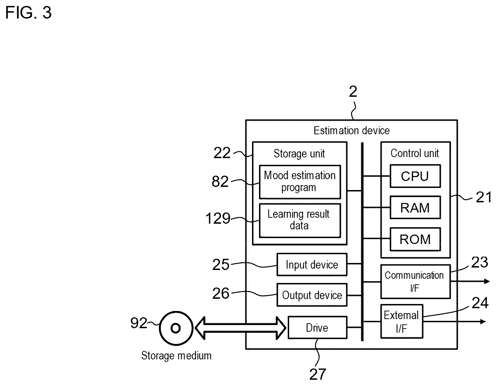

Fig. 2 schematically shows an example of a hardware configuration of the model generation device 1 according to the present embodiment. As illustrated in Fig. 2, the model generation device 1 according to the present embodiment is a computer to which a control unit 11, a storage unit 12, a communication interface 13, an external interface 14, an input device 15, an output device 16, and a drive 17 are electrically connected. In Fig. 2, the communication interface and the external interface are referred to as a "communication I/F" and an "external I/F", respectively. The same notation is used in Fig. 3 described later.

The control unit 11 includes a central processing unit (CPU) that is a hardware processor, a random access memory (RAM), a read only memory (ROM), and the like, and is configured to execute information processing based on a program and various data. The CPU is an example of a processor resource. As the processor resource, a graphics processing unit (GPU) may be used instead of the CPU or together with the CPU. The storage unit 12 is an example of a memory resource, and includes, for example, a hard disk drive, a solid state drive, or the like. In the present embodiment, the storage unit 12 stores various types of information such as a model generation program 81, a plurality of training data sets 120, and learning result data 129.

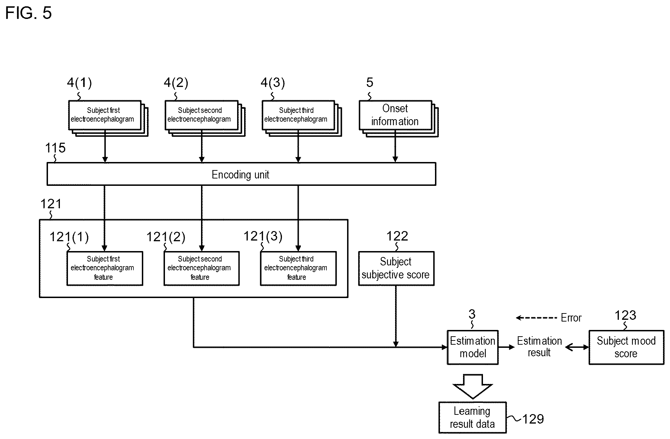

The model generation program 81 is a program for causing the model generation device 1 to execute information processing (fig. 5) described later related to machine learning of the estimation model 3 and the like, and includes a series of commands of the information processing. The plurality of training data sets 120 are used for machine learning of the estimation model 3. The learning result data 129 indicates information regarding a result of the machine learning (in the present embodiment, the trained estimation model 3 generated by machine learning). In the present embodiment, the learning result data 129 is generated as a result of executing the model generation program 81.

The communication interface 13 is, for example, a wired local area network (LAN) module, a wireless LAN module, or the like, and is an interface for performing wired or wireless communication via a network. The model generation device 1 may execute data communication with another information processing device via a network using the communication interface 13. The external interface 14 is, for example, a universal serial bus (USB) port, a dedicated port, or the like, and is an interface for connecting to an external device. The type and the number of external interfaces 14 may be appropriately selected according to the type and the number of external devices to be connected. In a case where data such as the subject electroencephalogram 4 is acquired by a detection device such as an electroencephalograph, the model generation device 1 may be connected to a target detection device via at least one of the communication interface 13 and the external interface 14.

The input device 15 is, for example, a device for performing input such as a mouse and a keyboard. Furthermore, the output device 16 is, for example, a device for performing output, such as a display and a speaker. An operator such as a user can operate the model generation device 1 by using the input device 15 and the output device 16.

The drive 17 is, for example, a CD drive, a DVD drive, or the like, and is a drive device for reading various kinds of information such as a program stored in the storage medium 91. The storage medium 91 is a medium that accumulates information such as a stored program by electrical, magnetic, optical, mechanical, or chemical action so that a computer, other devices, a machine, or the like can read the various information such as the program. At least one of the model generation program 81 and the plurality of training data sets 120 may be stored in the storage medium 91. The model generation device 1 may acquire at least one of the model generation program 81 and the plurality of training data sets 120 from the storage medium 91. Note that, in Fig. 2, a disk-type storage medium such as a CD or a DVD is illustrated as an example of the storage medium 91. However, the type of the storage medium 91 is not limited to the disk type, and may be other than the disk type. Examples of the storage medium other than the disk type include a semiconductor memory such as a flash memory. The type of the drive 17 may be arbitrarily selected according to the type of the storage medium 91.

Note that, regarding a specific hardware configuration of the model generation device 1, it is possible to appropriately omit, replace, and add structural elements according to the embodiment. For example, processor resources may include multiple hardware processors. The hardware processor may include a microprocessor, a field-programmable gate array (FPGA), a digital signal processor (DSP), or the like. The storage unit 12 may include a RAM and a ROM included in the control unit 11. At least one of the communication interface 13, the external interface 14, the input device 15, the output device 16, and the drive 17 may be omitted. The model generation device 1 may include a plurality of computers. In this case, the hardware configurations of the computers may or may not be the same. In addition, the model generation device 1 may be a general-purpose server device, a personal computer (PC), or the like, in addition to an information processing device designed exclusively for a service to be provided.

### <Estimation device>

Fig. 3 schematically shows an example of a hardware configuration of the estimation device 2 according to the present embodiment. As illustrated in Fig. 3, the estimation device 2 according to the present embodiment is a computer to which a control unit 21, a storage unit 22, a communication interface 23, an external interface 24, an input device 25, an output device 26, and a drive 27 are electrically connected.

The control unit 21 to the drive 27 and the storage medium 92 of the estimation device 2 may be configured similarly to the control unit 11 to the drive 17 and the storage medium 91 of the model generation device 1, respectively. The control unit 21 includes a CPU that is a hardware processor, a RAM, a ROM, and the like, and is configured to execute various types of information processing based on a program and data. The control unit 21 may use a GPU instead of the CPU or together with the CPU. The storage unit 22 includes, for example, a hard disk drive, a solid state drive, or the like. The storage unit 22 stores various types of information such as the mood estimation program 82 and the learning result data 129.

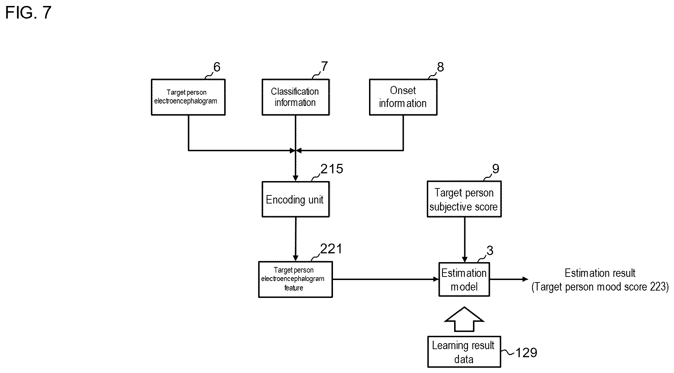

The mood estimation program 82 is a program for causing the estimation device 2 to execute information processing (fig. 7) to be described later that executes a predetermined estimation task for at least the target person electroencephalogram feature 221 using a trained machine learning model (in the present embodiment, the estimation model 3). The mood estimation program 82 includes a series of commands for the information processing. At least one of the mood estimation program 82 and the learning result data 129 may be stored in the storage medium 92. Further, the estimation device 2 may acquire at least one of the mood estimation program 82 and the learning result data 129 from the storage medium 92.

Note that, regarding a specific hardware configuration of the estimation device 2, structural elements can be omitted, replaced, and added as appropriate according to the embodiment. For example, the processor resource of the estimation device 2 may include a plurality of hardware processors. The hardware processor may be configured by a microprocessor, an FPGA, a DSP, or the like. The storage unit 22 may include a RAM and a ROM included in the control unit 21. At least one of the communication interface 23, the external interface 24, the input device 25, the output device 26, and the drive 27 may be omitted. The estimation device 2 may include a plurality of computers. In this case, the hardware configurations of the computers may or may not be the same. Furthermore, the estimation device 2 may be a general-purpose server device, a general-purpose PC, or the like, in addition to an information processing device designed exclusively for a service to be provided.

### [Software configuration]

### <Model generation device>

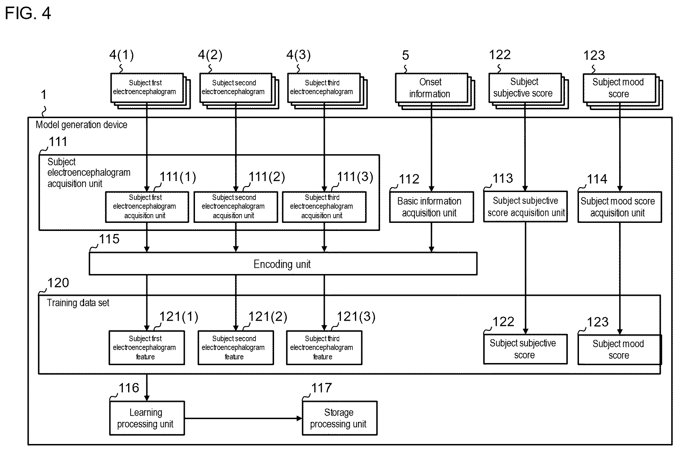

Fig. 4 schematically shows an example of a software configuration of the model generation device 1 according to the present embodiment. The control unit 11 of the model generation device 1 deploys the model generation program 81 stored in the storage unit 12 in the RAM. Then, the control unit 11 controls each structural element by interpreting and executing an instruction included in the model generation program 81 deployed in the RAM by the CPU. As a result, the model generation device 1 according to the present embodiment operates as a computer including a subject electroencephalogram acquisition unit 111, a basic information acquisition unit 112, a subject subjective score acquisition unit 113, a subject mood score acquisition unit 114, an encoding unit 115, a learning processing unit 116, and a storage processing unit 117 illustrated in Fig. 4 as software modules. That is, in the present embodiment, each software module of the model generation device 1 is realized by the control unit 11 (CPU).

The subject electroencephalogram acquisition unit 111 acquires a subject electroencephalogram 4, which is an electroencephalogram of the learning subject measured when listening to a voice uttering a sentence. The subject electroencephalogram acquisition unit 111 acquires the subject electroencephalogram 4 of each of a plurality of learning subjects ("135 learning subjects" in the present embodiment).

In the present embodiment, the subject electroencephalogram acquisition unit 111 acquires a plurality of subject electroencephalograms 4 of each learning subject for each category of "Ng", "Nt", and "Ps". As illustrated in Fig. 4, the subject electroencephalogram acquisition unit 111 may include a subject first electroencephalogram acquisition unit 111 (1), a subject second electroencephalogram acquisition unit 111 (2), and a subject third electroencephalogram acquisition unit 111 (3). The subject first electroencephalogram acquisition unit 111 (1) acquires, as a plurality of (for example, five) subject first electroencephalograms 4(1) of each learning subject, electroencephalograms when each learning subject is listening to a voice uttering each of a plurality of (for example, five) sentences classified into the category "Ng". Each of the plurality of subject first electroencephalograms 4(1) of each learning subject is an electroencephalogram of each learning subject listening to a voice uttering a sentence classified into the category "Ng". The subject second electroencephalogram acquisition unit 111 (2) acquires, as a plurality of (for example, five) subject second electroencephalograms 4(2) of each learning subject, electroencephalograms when each learning subject is listening to a voice uttering each of a plurality of (for example, five) sentences classified into the category "Nt". Each of the plurality of subject second electroencephalograms 4(2) of each learning subject is an electroencephalogram of each learning subject listening to a voice uttering a sentence classified into the category "Nt". The subject third electroencephalogram acquisition unit 111 (3) acquires, as a plurality of (for example, five) subject third electroencephalograms 4(3) of each learning subject, electroencephalograms when each learning subject is listening to a voice uttering each of a plurality of (for example, five) sentences classified into the category "Ps". Each of the plurality of subject third electroencephalograms 4(3) of each learning subject is an electroencephalogram of each learning subject listening to a voice uttering a sentence classified into the category "Ps". The subject electroencephalogram acquisition unit 111 notifies the encoding unit 115 of the acquired plurality of subject first electroencephalograms 4(1) of each learning subject, the acquired plurality of subject second electroencephalograms 4(2) of each learning subject, and the acquired plurality of subject third electroencephalograms 4(3) of each learning subject.

The basic information acquisition unit 112 acquires basic information used for generating the electroencephalogram feature Fw from the electroencephalogram of the person when listening to the voice uttering the sentence. The basic information acquired by the basic information acquisition unit 112 is at least one of onset information 5 and envelope information. The onset information 5 is information indicating a start point of each word included in the sentence that the learning subject was listening to as a voice. The envelope information is information indicating a voice envelope of a voice that a person is listening to. In the present embodiment, the basic information acquisition unit 112 acquires, as the basic information, the onset information 5 indicating the start point of each word included in the sentence that the learning subject was listening to as a voice. The basic information acquisition unit 112 notifies the encoding unit 115 of the acquired onset information 5.

The subject subjective score acquisition unit 113 acquires, as the subjective score Ss of each learning subject, the subject subjective score 122 indicating the subjective evaluation felt by each learning subject for the sentence after listening to the voice uttering the sentence. The subject subjective score acquisition unit 113 stores the acquired subject subjective score 122 of each learning subject in the training data set 120 of the storage unit 12.

The subject mood score acquisition unit 114 acquires the subject mood score 123 indicating the level of the depressed mood of each learning subject as the mood score Sm of each learning subject. The subject mood score acquisition unit 114 stores the acquired subject mood score 123 of each learning subject in the training data set 120 of the storage unit 12.

The encoding unit 115 generates an electroencephalogram feature Fw from an electroencephalogram of a person when listening to a voice uttering a sentence. In the present embodiment, the encoding unit 115 generates the subject electroencephalogram feature 121 from the electroencephalogram (that is, subject electroencephalogram 4) of the learning subject when listening to the voice uttering the sentence.

In the present embodiment, the encoding unit 115 statistically processes (for example, averages) a plurality of subject electroencephalograms 4 of each learning subject when listening to voice uttering sentences respectively classified into the categories of "Ng", "Nt", and "Ps", and generates the subject electroencephalogram features 121 of each learning subject corresponding to the respective categories of "Ng", "Nt", and "Ps". Specifically, the encoding unit 115 generates the subject first electroencephalogram feature 121(1), the subject second electroencephalogram feature 121(2), and the subject third electroencephalogram feature 121(3) of each learning subject as the first electroencephalogram feature Fw(1), the second electroencephalogram feature Fw(2), and the third electroencephalogram feature Fw(3) of each learning subject.

Fig. 5 schematically shows an example of each process of the feature generation processing and the learning processing executed by the model generation device 1 in the present embodiment. As illustrated in Fig. 5, the encoding unit 115 statistically processes a plurality of subject first electroencephalograms 4(1) of each learning subject to generate the subject first electroencephalogram features 121(1) of each learning subject as the subject electroencephalogram features 121 corresponding to the category of "Ng" of each learning subject. Furthermore, the encoding unit 115 statistically processes the plurality of subject second electroencephalograms 4(2) of each learning subject to generate the subject second electroencephalogram features 121(2) of each learning subject as the subject electroencephalogram features 121 corresponding to the category of "Nt" of each learning subject. Furthermore, the encoding unit 115 statistically processes the plurality of subject third electroencephalograms 4(3) of each learning subject to generate the subject third electroencephalogram features 121(3) of each learning subject as the subject electroencephalogram features 121 corresponding to the category of "Ps" of each learning subject.

In particular, the encoding unit 115 statistically processes (for example, averages) the plurality of "component features Ifa in the 'electroencephalogram response to a word' of each learning subject" for each category of "Ng", "Nt", and "Ps", and generates the subject electroencephalogram feature 121 of each learning subject for each category of "Ng", "Nt", and "Ps".

For example, the encoding unit 115 generates, based on (A) the plurality of subject first electroencephalograms 4(1) of each learning subject, and (B) the onset information 5 indicating the start point of each word included in each of the plurality of sentences classified as "Ng" that each learning subject is listening, (C) the component feature Ifa (1) of the "electroencephalogram response to a word" of each learning subject for each of the plurality of sentences. Then, the encoding unit 115 statistically processes the plurality of component features Ifa (1), which are the "component features Ifa of the 'electroencephalogram response to a word' of each learning subject" for the sentence each classified as "Ng", to generate the subject first electroencephalogram feature 121(1) of each learning subject.

Similarly, the encoding unit 115 generates, based on (A) the plurality of subject second electroencephalograms 4(2) of each learning subject, and (B) the onset information 5 indicating the start point of each word included in each of the plurality of sentences classified as "Nt" that each learning subject is listening, (C) the component feature Ifa (2) of the "electroencephalogram response to a word" of each learning subject for each of the plurality of sentences. Then, the encoding unit 115 statistically processes the plurality of component features Ifa (2), which are the "component features Ifa of the 'electroencephalogram response to a word' of each learning subject" for the sentence each classified as "Nt", to generate the subject second electroencephalogram feature 121(2) of each learning subject.

In addition, the encoding unit 115 generates, based on (A) the plurality of subject third electroencephalograms 4(3) of each learning subject, and (B) the onset information 5 indicating the start point of each word included in each of the plurality of sentences classified as "Ps" that each learning subject is listening, (C) the component feature Ifa (3) of the "electroencephalogram response to a word" of each learning subject for each of the plurality of sentences. Then, the encoding unit 115 statistically processes the plurality of component features Ifa (3), which are the "component features Ifa of the 'electroencephalogram response to a word' of each learning subject" for the sentence each classified as "Ps", to generate the subject third electroencephalogram feature 121(3) of each learning subject.

The encoding unit 115 stores the generated subject first electroencephalogram feature 121(1), subject second electroencephalogram feature 121(2), and subject third electroencephalogram feature 121(3) of each learning subject in the training data set 120 of the storage unit 12.

The learning processing unit 116 performs machine learning of the estimation model 3 using the plurality of training data sets 120 stored in the storage unit 12. As described above, the encoding unit 115 stores the subject electroencephalogram feature 121 of each learning subject in each training data set 120, and the subject mood score acquisition unit 114 stores the subject mood score 123 of each learning subject in each training data set 120. Therefore, each training data set 120 is formed by associating the subject mood score 123 of each learning subject with at least the subject electroencephalogram feature 121 of each learning subject.

The machine learning performed by the learning processing unit 116 includes at least the training steps described below. That is, a training step of training the estimation model 3 so that the subjective score Ss estimated by the estimation model 3 when the subject electroencephalogram feature 121 is received as an input matches the subject mood score 123, for each training data set 120 is included.

In the present embodiment, in each training data set 120, the subject subjective score 122 of each learning subject is further stored by the subject subjective score acquisition unit 113. That is, in each training data set 120, the subject mood score 123 of each learning subject is associated with the subject electroencephalogram feature 121 and the subject subjective score 122 of each learning subject. Therefore, the machine learning performed by the learning processing unit 116 in the present embodiment includes the training steps described below. That is, a training step of training the estimation model 3 so that the subjective score Ss estimated by the estimation model 3 when the subject electroencephalogram feature 121 and the subject subjective score 122 are input matches the subject mood score 123, for each training data set 120 is included.

In particular, in each training data set 120 according to the present embodiment, the subject first electroencephalogram feature 121(1), the subject second electroencephalogram feature 121(2), and the subject third electroencephalogram feature 121(3) are stored as the subject electroencephalogram feature 121 of each learning subject by the encoding unit 115. That is, in each training data set 120, the subject mood score 123 of each learning subject is associated with the subject first electroencephalogram feature 121(1), the subject second electroencephalogram feature 121(2), the subject third electroencephalogram feature 121(3), and the subject subjective score 122 of each learning subject.

Therefore, the machine learning performed by the learning processing unit 116 in the present embodiment includes the first training step, second training step, and third training step described below. In the first training step, the estimation model 3 is trained so that the subjective score Ss estimated by the estimation model 3 when at least the first electroencephalogram feature Fw(1) (the subject first electroencephalogram feature 121(1)) of the learning subject is input matches the subject mood score 123. In the second training step, the estimation model 3 is trained so that the subjective score Ss estimated by the estimation model 3 when at least the second electroencephalogram feature Fw(2) of the learning subject (the subject second electroencephalogram feature 121(2)) is input matches the subject mood score 123. In the third training step, the estimation model 3 is trained so that the subjective score Ss estimated by the estimation model 3 when at least the third electroencephalogram feature Fw(3) (the subject third electroencephalogram feature 121(3)) of the learning subject is input matches the subject mood score 123.

Specifically, as illustrated in Fig. 5, in the first training step, the estimation model 3 is trained so that the subjective score Ss estimated by the estimation model 3 when the subject first electroencephalogram feature 121(1) and the subject subjective score 122 are input matches the subject mood score 123. In the second training step, the estimation model 3 is trained so that the subjective score Ss estimated by the estimation model 3 when the subject second electroencephalogram feature 121(2) and the subject subjective score 122 are input matches the subject mood score 123. In the third training step, the estimation model 3 is trained so that the subjective score Ss estimated by the estimation model 3 when the subject third electroencephalogram feature 121(3) and the subject subjective score 122 are input matches the subject mood score 123. By executing each training step described above, the trained estimation model 3 is generated. A value of an operation parameter of the estimation model 3 is adjusted by each training step of the machine learning.

The storage processing unit 117 generates information indicating the structure of the trained estimation model 3 and the value of the operation parameter as the learning result data 129. The structure may be specified by, for example, the number of layers from the input layer to the output layer, the type of each layer, the number of neurons included in each layer, a connection relationship between neurons in adjacent layers, and the like in the neural network. In a case where the structure of the model (the estimation model 3) is shared in the mood estimation system 100, information on this structure may be omitted from the learning result data 129. Furthermore, information that is not used in the usage scene may be omitted from the learning result data 129. The storage processing unit 117 stores the generated learning result data 129 in a predetermined storage area (the storage unit 12 in the present embodiment).

### <Estimation device>

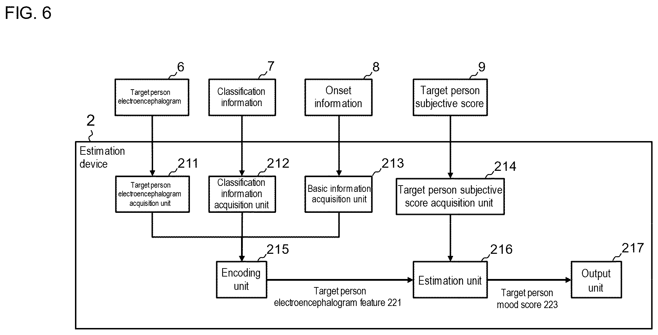

Fig. 6 schematically shows an example of a software configuration of the estimation device 2 according to the present embodiment. The controller 21 of the estimation device 2 deploys the mood estimation program 82 stored in the storage unit 22 in the RAM. The controller 21 causes the CPU to interpret and execute commands included in mood estimation program 82 deployed in the RAM to control the respective structural element. As a result, the estimation device 2 according to the present embodiment operates as a computer including a target person electroencephalogram acquisition unit 211, a classification information acquisition unit 212, a basic information acquisition unit 213, a target person subjective score acquisition unit 214, an encoding unit 215, an estimation unit 216, and an output unit 217 illustrated in Fig. 6 as software modules. That is, in the present embodiment, each software module of the estimation device 2 is realized by the control unit 21(CPU) similarly to the model generation device 1.

The target person electroencephalogram acquisition unit 211 is an example of a software module that executes the "target person electroencephalogram acquisition step" of the present invention. The target person electroencephalogram acquisition unit 211 acquires a target person electroencephalogram 6 which is an electroencephalogram of the target person measured when listening to a voice uttering a sentence. The target person electroencephalogram acquisition unit 211 notifies the encoding unit 215 of the acquired target person electroencephalogram 6.

The classification information acquisition unit 212 is an example of a software module that executes the "classification information acquisition step" of the present invention. The classification information acquisition unit 212 acquires classification information 7 indicating into which of at least three categories of "Ng", "Nt", and "Ps" a sentence the subject is listening to as a voice is classified. The classification information acquisition unit 212 notifies the encoding unit 215 of the acquired classification information 7.

The basic information acquisition unit 213 is an example of a software module that executes at least one of the "onset information acquisition step" and the "envelope information acquisition step" of the present invention. In the present embodiment, similarly to the basic information acquisition unit 112, the basic information acquisition unit 213 acquires the basic information, and specifically, acquires onset information 8 indicating a start point of each word included in the sentence that the subject was listening to as a voice. The basic information acquisition unit 213 notifies the encoding unit 215 of the obtained onset information 8. Note that, similarly to the basic information acquisition unit 112, the basic information acquisition unit 213 may acquire envelope information indicating a voice envelope of a voice that the subject is listening to, instead of the onset information 8 or together with the onset information 8.

The target person subjective score acquisition unit 214 is an example of a software module that executes the "target person subjective score acquisition step" of the present invention. The target person subjective score acquisition unit 214 acquires, as the subjective score Ss of the target person, the target person subjective score 9 indicating the subjective evaluation felt by the target person for the sentence after listening to the voice uttering the sentence. The target person subjective score acquisition unit 214 notifies the estimation unit 216 of the acquired target person subjective score 9.

The encoding unit 215 is an example of a software module that executes the "electroencephalogram encoding step" of the present invention. The encoding unit 215 generates the target person electroencephalogram feature 221 from the target person electroencephalogram 6. In the present embodiment, the encoding unit 215 generates the target person electroencephalogram feature 221(x) corresponding to the category (x) indicated by the classification information 7 based on the target person electroencephalogram 6 and the classification information 7. The "x" is an integer of "1" or more, and for example, category (1) corresponds to "Ng", category (2) corresponds to "Nt", and category (3) corresponds to "Ps".

Specifically, in a case where the classification information 7 indicates that the sentence that the subject is listening to as a voice is classified as "Ng", the encoding unit 215 generates the target person electroencephalogram feature 221(1) as the first electroencephalogram feature Fw(1) of the target person. Furthermore, in a case where the classification information 7 indicates that the sentence that the target person is listening to as a voice is classified as "Nt", the encoding unit 215 generates the target person electroencephalogram feature 221(2) as the second electroencephalogram feature Fw(2) of the target person. Furthermore, in a case where the classification information 7 indicates that the sentence that the target person is listening to as a voice is classified as "Ps", the encoding unit 215 generates the target person electroencephalogram feature 221(3) as the third electroencephalogram feature Fw(3) of the target person. In the following description, when the target person electroencephalogram features 221(1), 221(2), and 221(3) are not particularly distinguished, they may be referred to as "target person electroencephalogram features 221".

The encoding unit 215 generates the component feature Ifa in the "electroencephalogram response to a word" of the target person as the electroencephalogram feature Fw based on the target person electroencephalogram 6 and the onset information 8. In the present embodiment, the encoding unit 215 can generate the component feature Ifa in the "electroencephalogram response to a word" of the target person for each category of "Ng", "Nt", and "Ps", that is, generate the electroencephalogram feature Fw of the target person for each category of "Ng", "Nt", and "Ps". Specifically, the encoding unit 215 generates the component feature Ifa (x) in "electroencephalogram response to a word included in a sentence classified into category (x) indicated by the classification information 7" of the target person as the target person electroencephalogram feature 221(x) corresponding to the category (x) indicated by the classification information 7 based on the target person electroencephalogram 6, the classification information 7, and the onset information 8.

For example, in a case where the classification information 7 indicates that a sentence that the target person is listening to as a voice is classified into "Ng", the encoding unit 215 generates the component feature Ifa (1) in "electroencephalogram response to a word included in a sentence classified into 'Ng'" of the target person as the target person electroencephalogram feature 221(1) corresponding to "Ng". Furthermore, in a case where the classification information 7 indicates that the sentence that the target person is listening to as a voice is classified into "Nt", the encoding unit 215 generates the component feature Ifa (2) in the "electroencephalogram response to a word included in a sentence classified into 'Nt'" of the target person as the target person electroencephalogram feature 221(2) corresponding to "Nt". Furthermore, in a case where the classification information 7 indicates that the sentence that the target person is listening to as a voice is classified as "Ps", the encoding unit 215 generates the component feature Ifa (3) in the "electroencephalogram response to a word included in a sentence classified as 'Ps'" of the target person as the target person electroencephalogram feature 221(3) corresponding to "Ps".

The estimation unit 216 is an example of a software module that executes the "estimation step" of the present invention. The estimation unit 216 uses the trained machine learning model (that is, the estimation model 3) generated by the model generation device 1 to execute the estimation task for at least the target person electroencephalogram feature 221, that is, to estimate the target person mood score 223. The estimation unit 216 includes the trained estimation model 3 generated by the model generation device 1 by holding the learning result data 129. The estimation unit 216 estimates the target person mood score 223 from at least the target person electroencephalogram feature 221 using the trained estimation model 3.

In the present embodiment, the target person electroencephalogram feature 221 is the target person electroencephalogram feature 221(x) corresponding to the category (x) indicated by the classification information 7, and is particularly the component feature Ifa (x) in the "electroencephalogram response to a word included in a sentence classified into category (x) indicated by classification information 7" of the target person. As described above, the trained estimation model 3 is trained so that the subjective score Ss estimated by the estimation model 3 when the subject electroencephalogram feature 121(subject first electroencephalogram feature 121(1), subject second electroencephalogram feature 121(2), subject third electroencephalogram feature 121(3)) for each category of "Ng", "Nt", and "Ps" is input matches the subject mood score 123. Then, the subject first electroencephalogram feature 121(1) is an average of the component features Ifa (1) in the "electroencephalogram response to a word included in a sentence classified as 'Ng'" of each learning subject. In addition, the subject second electroencephalogram feature 121(2) is an average of the component features Ifa (2) in the "electroencephalogram response to a word included in a sentence classified as 'Nt'" of each learning subject. Furthermore, the subject third electroencephalogram feature 121(3) is an average of the component features Ifa (3) in the "electroencephalogram response to a word included in a sentence classified as 'Ps'" of each learning subject. Therefore, the estimation unit 216 can estimate the target person mood score 223 by giving the component feature Ifa (x) in "electroencephalogram response to a word included in a sentence classified into category (x) indicated by classification information 7" of the target person to the trained estimation model 3.

In particular, the estimation unit 216 estimates the target person mood score 223 based on the target person electroencephalogram feature 221 and the target person subjective score 9. As described above, the trained estimation model 3 is trained so that the subjective score Ss estimated by the estimation model 3 when the subject electroencephalogram feature 121 and the subject subjective score 122 are input matches the subject mood score 123. Therefore, the estimation unit 216 can estimate the target person mood score 223 by giving the target person electroencephalogram feature 221 and the target person subjective score 9 to the trained estimation model 3.

For example, the estimation unit 216 estimates the target person mood score 223 by giving the component feature Ifa (1) (the target person electroencephalogram feature 221(1) corresponding to "Ng") and the target person subjective score 9 in the electroencephalogram response to a word included in a sentence classified as "Ng" of the target person to the trained estimation model 3. In addition, the estimation unit 216 estimates the target person mood score 223 by giving the component feature Ifa (2) (the target person electroencephalogram feature 221(2) corresponding to "Nt") and the target person subjective score 9 in the electroencephalogram response to a word included in a sentence classified as "Nt" of the target person to the trained estimation model 3. Furthermore, the estimation unit 216 estimates the target person mood score 223 by giving the component features Ifa (3) (the target person electroencephalogram feature 221(3) corresponding to "Ps") and the target person subjective score 9 in the electroencephalogram response to a word included in a sentence classified as "Ps" of the target person to the trained estimation model 3.

Fig. 7 schematically shows an example of each process of the feature generation processing and the mood estimation processing executed by the estimation device 2. As illustrated in Fig. 7, the encoding unit 215 generates the component feature Ifa (x) in the "electroencephalogram response to a word included in a sentence classified into category (x) indicated by the classification information 7 (the target person electroencephalogram feature 221(x) corresponding to the category (x) indicated by the classification information 7)" of the target person based on the target person electroencephalogram 6, the classification information 7, and the onset information 8. That is, the encoding unit 215 generates any one of the target person electroencephalogram feature 221(1) that is the first electroencephalogram feature Fw(1) of the target person, the target person electroencephalogram feature 221(2) that is the second electroencephalogram feature Fw(2) of the target person, and the target person electroencephalogram feature 221(3) that is the third electroencephalogram feature Fw(3) of the target person according to the category indicated by the classification information 7. Then, the trained estimation model 3 estimates (outputs) the target person mood score 223 based on the target person electroencephalogram feature 221(in particular, the target person electroencephalogram feature 221(x) corresponding to the category (x) indicated by the classification information 7) and the target person subjective score 9.

The output unit 217 is an example of a software module that executes the "output step" of the present invention. The output unit 217 outputs information corresponding to the target person mood score 223 estimated in the estimation step to the target person.

In this configuration, the estimation device 2 outputs (for example, notifies) information corresponding to the target person mood score 223 to the target person. The information corresponding to the target person mood score 223 may be the target person mood score 223 itself. In addition, the information corresponding to the target person mood score 223 may be information indicating the level of the depressed mood of the target person indicated by the target person mood score 223. Furthermore, the information corresponding to the target person mood score 223 may be information including advice to the target person, corresponding to the target person mood score 223. For example, by outputting the target person mood score 223 to the target person, the estimation device 2 can allow the target person aware of a state of mind such as depression of his/her own mood. For example, the estimation device 2 outputs, to the target person, information including advice to the target person, corresponding to the target person mood score 223, thereby urging the target person to take an action for maintaining mental health soundness, such as blocking information that puts a heavy load on the mental.

In a case where the target person mood score 223 indicates that the target person has a high level of depressed mood, the information corresponding to the target person mood score 223 may be information for relaxing the target person, for example, music, video, or the like for relaxing the target person. The estimation device 2 can output information according to the level of the depressed mood of the target person indicated by the target person mood score 223 to the target person as "information corresponding to target person mood score 223".

In particular, the estimation device 2 estimates the target person mood score 223 from the electroencephalogram of the target person when the target person is listening to a voice uttering a sentence such as a news voice daily heard by the target person, instead of the unusual (special) sound as described in NPL 1. Therefore, the estimation device 2 estimates the target person mood score 223 from the electroencephalogram of the target person with respect to the daily speech information, and outputs information corresponding to the estimated target person mood score 223 to the target person, so that the target person can be made aware of his/her own state of mind, urged to take an action to keep mental health sound, and relaxed.

### <Others>

The software modules of the model generation device 1 and the estimation device 2 will be described in detail in an operation example to be described later. In the present embodiment, an example in which each software module of the model generation device 1 and the estimation device 2 is realized by a general-purpose CPU has been described. However, some or all of the software modules may be realized by one or a plurality of dedicated processors. For example, some or all of the software modules may be processed by a graphics processing unit. Furthermore, regarding the software configuration of each of the model generation device 1 and the estimation device 2, omission, replacement, and addition of the software module may be appropriately performed according to the embodiment.

### §3 Operation example

### [Model generation device]

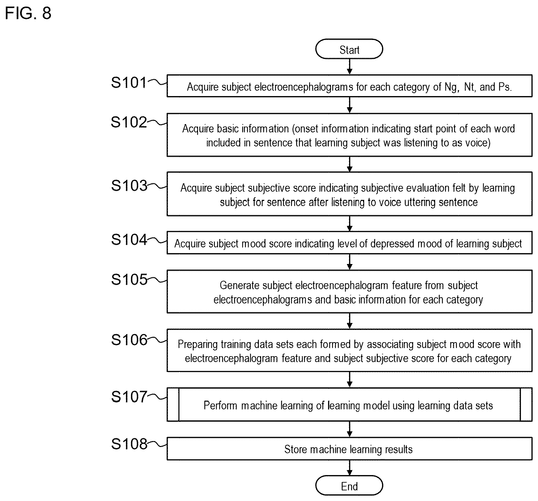

Fig. 8 is a flowchart illustrating an example of a processing procedure of the model generation device 1 according to the present embodiment. However, the processing procedure described below is merely an example, and each step may be changed as much as possible. Furthermore, with respect to the processing procedure described below, it is possible to appropriately omit, replace, and add steps according to the embodiment.

### (Step S101)

In step S101, the control unit 11 operates as the subject electroencephalogram acquisition unit 111, and acquires a plurality of subject electroencephalograms 4 of each learning subject for each category of "Ng", "Nt", and "Ps". That is, the control unit 11 acquires a plurality of subject first electroencephalograms 4(1), subject second electroencephalograms 4(2), and subject third electroencephalograms 4(3) of each learning subject.

### (Step S102)

In step S102, the control unit 11 operates as the basic information acquisition unit 112, and acquires, in the present embodiment, the onset information 5 indicating the start point of time of each word included in the sentence that the learning subject was listening to as a voice.

### (Step S103)

In step S103, the control unit 11 operates as the subject subjective score acquisition unit 113, and acquires the subject subjective score 122 indicating the subjective evaluation felt by each learning subject for the sentence after listening to the voice uttering the sentence. The control unit 11 stores the acquired subject subjective score 122 of each learning subject in the training data set 120 of the storage unit 12.

### (Step S104)

In step S104, the control unit 11 operates as the subject mood score acquisition unit 114, and acquires the subject mood score 123 indicating the level of the depressed mood of each learning subject. The control unit 11 stores the acquired subject mood score 123 of each learning subject in the training data set 120 of the storage unit 12.

### (Step S105)

In step S105, the control unit 11 operates as the encoding unit 115, and generates the subject electroencephalogram feature 121 based on the plurality of subject electroencephalograms 4 and the basic information (the onset information 5) for each category of "Ng", "Nt", and "Ps". That is, the control unit 11 generates the component feature Ifa (1) of the "electroencephalogram response to a word included in a sentence classified as 'Ng'" of each learning subject as the subject first electroencephalogram feature 121(1) of each learning subject based on the plurality of subject first electroencephalograms 4(1) of each learning subject and the onset information 5. In addition, the control unit 11 generates the component feature Ifa (2) of the "electroencephalogram response to a word included in a sentence classified as 'Nt'" of each learning subject as the subject second electroencephalogram feature 121(2) of each learning subject based on the plurality of subject second electroencephalograms 4(2) of each learning subject and the onset information 5. Furthermore, the control unit 11 generates the component feature Ifa (3) of the "electroencephalogram response to a word included in a sentence classified as 'Ps'" of each learning subject as the subject third electroencephalogram feature 121(3) of each learning subject based on the plurality of subject third electroencephalograms 4(3) of each learning subject and the onset information 5.

### (Step S106)

In step S106, the control unit 11 operates as the encoding unit 115, and prepares a plurality of training data sets 120 each formed by associating the subject mood score 123 with "the subject electroencephalogram feature 121 and the subject subjective score 122 for each category of 'Ng', 'Nt', and 'Ps'". As described above, in S103, the subject subjective score 122 of each learning subject is stored in each training data set 120. In addition, in S104, the subject mood score 123 of each learning subject is stored in each training data set 120. Then, in step S106, the control unit 11 stores the subject first electroencephalogram feature 121(1), the subject second electroencephalogram feature 121(2), and the subject third electroencephalogram feature 121(3) of each learning subject generated in step S105 in each training data set 120. As a result, a plurality of training data sets 120 in which the subject mood score 123 of each learning subject is associated with the subject electroencephalogram features 121(the subject first electroencephalogram feature 121(1), the subject second electroencephalogram feature 121(2), and the subject third electroencephalogram feature 121(3)) and the subject subjective score 122 of each learning subject are prepared.

### (Step S107)

In step S107, the control unit 11 operates as the learning processing unit 116, and performs machine learning of the learning model (that is, the estimation model 3) using the plurality of training data sets 120. Performing the machine learning includes a training step of training the estimation model 3 so that, for each of the plurality of training data sets 120, the subjective score Ss estimated by the estimation model 3 when at least the subject electroencephalogram feature 121 is received as an input matches the subject mood score 123. Performing machine learning in the present embodiment includes a training step of training the estimation model 3 so that, for each training data set 120, the subjective score Ss estimated by the estimation model 3 when the subject electroencephalogram features 121(the subject first electroencephalogram feature 121(1), the subject second electroencephalogram feature 121(2) the subject third electroencephalogram feature 121(3)) and the subject subjective score 122 are input matches the subject mood score 123.

Specifically, performing the machine learning includes the first training step, second training step, and third training step as described below. In the first training step, the estimation model 3 is trained so that the subjective score Ss estimated by the estimation model 3 when the subject first electroencephalogram feature 121(1) and the subject subjective score 122 are input matches the subject mood score 123. In the second training step, the estimation model 3 is trained so that the subjective score Ss estimated by the estimation model 3 when the subject second electroencephalogram feature 121(2) and the subject subjective score 122 are input matches the subject mood score 123. In the third training step, the estimation model 3 is trained so that the subjective score Ss estimated by the estimation model 3 when the subject third electroencephalogram feature 121(3) and the subject subjective score 122 are input matches the subject mood score 123.

A neural network constituting the estimation model 3 to be processed by the machine learning may be appropriately prepared. The structure (for example, the number of layers, the number of neurons included in each layer, a connection relationship between neurons in adjacent layers, and the like) of the estimation model 3, an initial value of the weight of the connection between each neuron, and an initial value of a threshold of each neuron may be given by a template or by an input of an operator. Furthermore, in a case of performing relearning, the control unit 11 may prepare the estimation model 3 based on learning result data obtained by performing the past machine learning. For the training processing of the machine learning, for example, a batch gradient descent method, a stochastic gradient descent method, a mini-batch gradient descent method, or the like may be used.

For example, the control unit 11 calculates, for each training data set 120, an error between the output value obtained from the estimation model 3 and the "subject mood score 123" that is the correct data. The control unit 11 calculates the error of the value of each operation parameter (a weight of connection between neurons, a threshold value of each neuron, and the like) of the estimation model 3 using the calculated gradient of error by, for example, back propagation. The control unit 11 updates the value of each operation parameter of the estimation model 3 based on each calculated error. The degree of updating the value of each operation parameter may be adjusted by a learning rate. The learning rate may be given by designation of an operator or may be given as a setting value in a program.

The control unit 11 adjusts the values of the operation parameters of the estimation model 3 so that the sum of the calculated errors is reduced by the series of update processing described above. For example, the control unit 11 may repeat the adjustment of the value of each operation parameter by the series of processing described above until a predetermined condition is satisfied so that the sum of the calculated errors, which is executed a specified number of times, is equal to or less than a threshold value. As a result, the control unit 11 can train the estimation model 3 so that the execution result of the estimation task obtained from the estimation model 3 matches the correct data (the subject mood score 123) by giving the subject electroencephalogram feature 121 and the subject subjective score 122 to the estimation model 3 for each training data set 120. When the training processing in step S107 is completed, the control unit 11 proceeds to the next step S108.

### (Step S108)

In step S108, the control unit 11 operates as the storage processing unit 117, and stores the result of the machine learning, that is, stores the learning result data 129 indicating the structure of the trained estimation model 3 and the value of the operation parameter in a predetermined storage area.

### [Estimation device]

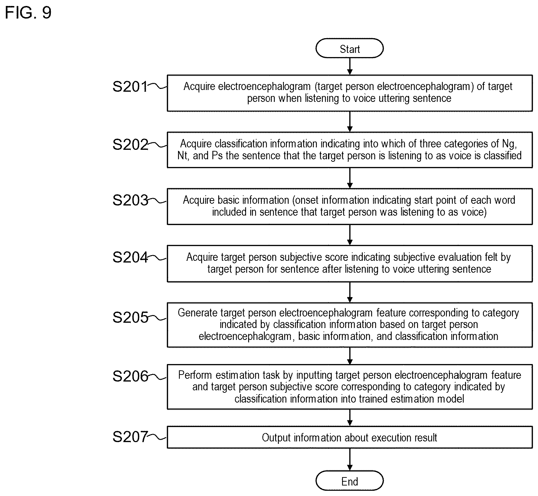

Fig. 9 is a flowchart illustrating an example of a processing procedure of the estimation device 2 according to the present embodiment. However, the processing procedure described below is merely an example, and each step may be changed as much as possible. Furthermore, with respect to the processing procedure described below, it is possible to appropriately omit, replace, and add steps according to the embodiment.

### (Step S201: Target person electroencephalogram acquisition step)

In step S201, the control unit 21 operates as the target person electroencephalogram acquisition unit 211, and acquires the electroencephalogram of the target person (the target person electroencephalogram 6) when the target person is listening to the voice uttering a sentence.

### (Step S202: Classification information acquisition step)

In step S202, the control unit 21 operates as the classification information acquisition unit 212, and acquires the classification information 7 indicating into which of at least three categories "Ng", "Nt", and "Ps" the sentence that the target person is listening to as a voice is classified.

### (Step S203: Onset information acquisition step)

In step S203, the control unit 21 operates as the basic information acquisition unit 213, and acquires, as the basic information, the onset information 8 indicating the start point of each word included in the sentence that the subject was listening to as a voice.

### (Step S204: Target person subjective score acquisition step)

In step S204, the control unit 21 operates as the target person subjective score acquisition unit 214, and acquires the target person subjective score 9 indicating the subjective evaluation felt by the target person for the sentence after listening to the voice uttering the sentence.

### (Step S205: Electroencephalogram encoding step)

In step S205, the control unit 21 operates as the encoding unit 215, and generates the target person electroencephalogram feature 221 from the target person electroencephalogram 6. In the present embodiment, the control unit 21 generates the target person electroencephalogram feature 221(x) corresponding to the category (x) indicated by the classification information 7 based on the target person electroencephalogram 6, the onset information 8, and the classification information 7. Specifically, the control unit 21 generates the component feature Ifa (x) in "electroencephalogram response to a word included in a sentence classified into category (x) indicated by classification information 7" of the target person as the target person electroencephalogram feature 221(x) corresponding to the category (x) indicated by the classification information 7.

### (Step S206, Estimation step)

In step S206, the control unit 21 operates as the estimation unit 216, and estimates the target person mood score 223 by inputting at least the target person electroencephalogram feature 221 to the trained estimation model 3. In the present embodiment, the control unit 21 inputs the "target person electroencephalogram feature 221(x) corresponding to the category (x) indicated by the classification information 7" generated in step S205 and the target person subjective score 9 to the trained estimation model 3, and executes the estimation task. That is, the control unit 21 inputs the "target person electroencephalogram feature 221(x) corresponding to the category (x) indicated by the classification information 7" and the target person subjective score 9 to the trained estimation model 3 to estimate (output) the target person mood score 223.

### (Step S207, Output step)

In step S207, the controller 21 operates as the output unit 217, and outputs the information regarding the execution result of the estimation task in step S206, that is, outputs the information corresponding to the target person mood score 223 estimated in the estimation step to the target person.

### [Features]

As described above, in the present embodiment, the mood estimation program 82 causes the estimation device 2 (the computer) to execute the target person electroencephalogram acquisition step (S201), the electroencephalogram encoding step (S205), and the estimation step (S206). In the target person electroencephalogram acquisition step, the estimation device 2 acquires the target person electroencephalogram 6 that is an electroencephalogram of the target person when the target person is listening to a voice uttering a sentence. In the electroencephalogram encoding step, the estimation device 2 generates an electroencephalogram feature Fw from an electroencephalogram of a person when listening to a voice uttering a sentence. In particular, in the electroencephalogram encoding step, the estimation device 2 generates the target person electroencephalogram feature 221 as the "target person electroencephalogram feature Fw" from the target person electroencephalogram 6. In the estimation step, the estimation device 2 inputs the target person electroencephalogram feature 221 to the trained estimation model 3 to estimate the target person mood score 223, which is the mood score Sm indicating the level of the depressed mood of the target person.

The estimation model 3 may be formed as, for example, a linear support vector machine that receives at least the electroencephalogram feature Fw as an input and estimates the mood score Sm indicating the level of person depressed mood. The estimation model 3 is generated by performing machine learning using a plurality of training data sets 120. Each of the plurality of training data sets 120 is formed by associating at least the subject electroencephalogram feature 121 that is the electroencephalogram feature Fw generated from the electroencephalogram of the learning subject (the subject electroencephalogram 4) when listening to the voice uttering the sentence with the subject mood score 123 that is the mood score Sm indicating the level of the depressed mood of the learning subject. Performing the machine learning includes a training step of training the estimation model 3 so that the mood score Sm estimated by the estimation model 3 when the subject electroencephalogram feature 121 is received as an input matches the subject mood score 123 for each of the plurality of training data sets 120.

In this configuration, the mood estimation program 82 causes the estimation device 2 to estimate the target person mood score 223 indicating the level of the depressed mood of the target person based on the target person electroencephalogram feature 221 generated from the target person electroencephalogram 6 of the target person when listening to the voice uttering the sentence. The mood estimation program 82 causes the estimation device 2 to estimate the target person mood score 223 by inputting the target person electroencephalogram feature 221 to the estimation model 3 generated by performing machine learning using the plurality of training data sets 120.

The training data set 120 is formed so that at least an electroencephalogram feature Fw (the subject electroencephalogram feature 121) generated from an electroencephalogram of a person (the learning subject) when listening to a voice uttering a sentence is associated with the mood score Sm (the subject mood score 123) indicating the level of depressed mood of the person. In addition, performing the machine learning includes a training step of training the estimation model 3 so that the mood score Sm estimated by the estimation model 3 from the subject electroencephalogram feature 121 matches the subject mood score 123 for each of the plurality of training data sets 120.

The present inventors verified the estimation accuracy for the estimation model 3 generated by performing machine learning using the plurality of training data sets 120 formed by associating the subject mood score 123 with the subject electroencephalogram feature 121, and obtained the verification results as described below. That is, it was confirmed that the area under the Roc-curve (AUC) of the estimation model 3 was "0.73". In addition, the estimation model 3 identified 66% of people with high level of depressed mood (people with a BDI score of 14 or more) as having high level of depressed mood. Therefore, the estimation device 2 can estimate the target person mood score 223 with high accuracy by inputting, to the estimation model 3, the target person electroencephalogram feature 221 generated from the electroencephalogram of the target person when listening to the "voice uttering a sentence" such as a news voice or a conversation voice.

In particular, the "voice uttering a sentence" such as a news voice or a conversation voice is not an unusual (special) sound described in NPL 1, but is a voice that the subject ordinarily hears, that is, daily voice information. Therefore, the estimation device 2 has an effect of being able to estimate the target person mood score 223 indicating the level of the depressed mood of the subject from the electroencephalogram (brain response) of the target person with respect to the daily speech information.

The mood estimation program 82 causes the estimation device 2 to generate the electroencephalogram feature Fw for each of at least three categories of "Ng", "Nt", and "Ps" in the electroencephalogram encoding step. That is, in the electroencephalogram encoding step, the estimation device 2 generates the electroencephalogram feature Fw corresponding to the category into which the sentence is classified as the above-described electroencephalogram feature Fw based on the electroencephalogram of the person when listening to the voice uttering the sentence classified into any of the at least three categories and the information indicating the category into which the sentence is classified.

In a case where the electroencephalogram feature Fw corresponding to a category into which the sentence that the person is listening to as a voice is classified is input as the electroencephalogram feature Fw, the estimation model 3 estimates the mood score Sm indicating the level of depressed mood of the person. For example, in a case where any one of the first electroencephalogram feature Fw(1) corresponding to the category of "Ng", the second electroencephalogram feature Fw(2) corresponding to the category of "Nt", and the third electroencephalogram feature Fw(3) corresponding to the category of "Ps" is input, the estimation model 3 estimates the mood score Sm.

The subject electroencephalogram feature 121 includes the subject first electroencephalogram feature 121(1), the subject second electroencephalogram feature 121(2), and the subject third electroencephalogram feature 121(3). The subject first electroencephalogram feature 121(1) is the first electroencephalogram feature Fw(1) generated by statistically processing (for example, averaging) a plurality of subject first electroencephalograms 4(1), each of which is the electroencephalogram of the learning subject when listening to a voice uttering a sentence classified into the category "Ng". The subject second electroencephalogram feature 121(2) is the second electroencephalogram feature Fw(2) generated by statistically processing a plurality of subject second electroencephalograms 4(2), each of which is the electroencephalogram of the learning subject when listening to a voice uttering a sentence classified into the category "Nt". The subject third electroencephalogram feature 121(3) is the third electroencephalogram feature Fw(3) generated by statistically processing a plurality of subject third electroencephalograms 4(3), each of which is the electroencephalogram of the learning subject when listening to a voice uttering a sentence classified into the category "Ps".

Performing machine learning includes the first training step, the second training step, and the third training step. In the first training step, the estimation model 3 is trained so that the mood score Sm estimated by the estimation model 3 when the subject first electroencephalogram feature 121(1) is input as the first electroencephalogram feature Fw(1) corresponding to the category of "Ng" matches the subject mood score 123 for each of the plurality of training data sets 120. In the second training step, the estimation model 3 is trained so that the mood score Sm estimated by the estimation model 3 when the subject second electroencephalogram feature 121(2) is input as the second electroencephalogram feature Fw(2) corresponding to the category of "Nt" matches the subject mood score 123 for each of the plurality of training data sets 120. In the third training step, the estimation model 3 is trained so that the mood score Sm estimated by the estimation model 3 when the subject third electroencephalogram feature 121(3) is input as the third electroencephalogram feature Fw(3) corresponding to the category of "Ps" matches the subject mood score 123 for each of the plurality of training data sets 120.

The mood estimation program 82 further causes the estimation device 2 to execute the classification information acquisition step (S202) of acquiring the classification information 7 indicating into which of the at least three categories the sentence the target person is listening to as a voice is classified. Then, the mood estimation program 82 causes the estimation device 2 to generate the target person electroencephalogram feature 221(x) corresponding to the category (x) indicated by the classification information 7 based on the target person electroencephalogram 6 of the target person when listening to the voice uttering the sentence classified into the category (x) indicated by the classification information 7 and the classification information 7 in the electroencephalogram encoding step. The mood estimation program 82 causes the estimation device 2 to execute processing of estimating the target person mood score 223 by inputting the target person electroencephalogram feature 221(x) generated in the electroencephalogram encoding step to the estimation model 3 as the electroencephalogram feature Fw corresponding to the category (x) indicated by the classification information 7 in the estimation step.

In this configuration, the training data set 120 is formed by associating the subject mood score 123 with the electroencephalogram feature Fw (the subject first electroencephalogram feature 121(1), the subject second electroencephalogram feature 121(2), the subject third electroencephalogram feature 121(3)) corresponding to each of at least three categories of "Ng", "Nt", and "Ps". Then, the estimation model 3 is generated by performing machine learning including the first training step, the second training step, and the third training step described above using the training data set 120. Therefore, the estimation model 3 can estimate the mood score Sm indicating the level of depressed mood of the person based on the electroencephalogram feature Fw (any one of the first electroencephalogram feature Fw(1), the second electroencephalogram feature Fw(2), and the third electroencephalogram feature Fw(3)) corresponding to each category of "Ng", "Nt", and "Ps".

Further, the estimation device 2 executes the classification information acquisition step of acquiring the classification information 7. The classification information 7 may be acquired from the outside of the estimation device 2. In addition, the estimation device 2 may generate the classification information 7 and acquire the generated classification information 7 in the above-described classification information acquisition step. As described above, the classification information 7 may be generated on a rule basis or may be generated on a model basis.

The estimation device 2 generates the target person electroencephalogram feature 221(x) corresponding to the category (x) indicated by the classification information 7 based on the target person electroencephalogram 6 of the target person when listening to the voice uttering the sentence classified into the category (x) indicated by the classification information 7. Then, the estimation device 2 inputs the target person electroencephalogram feature 221(x) to the estimation model 3 to estimate the target person mood score 223.

Therefore, the estimation device 2 can estimate the target person mood score 223 indicating the level of the depressed mood of the target person based on the electroencephalograms of the target person when listening to the voice uttering the sentence and the classification information 7 indicating whether the sentence is classified into any one of at least three categories of "Ng", "Nt", and "Ps".

The mood estimation program 82 may cause the estimation device 2 to generate, as the electroencephalogram feature Fw, the component feature Ifa in the "electroencephalogram response to a word included in a sentence" of the person from the electroencephalogram of the person when listening to the voice uttering the sentence in the electroencephalogram encoding step. For example, the estimation device 2 (in particular, encoding unit 215) generates the component feature Ifa in the "electroencephalogram response to a word" of the person as the electroencephalogram feature Fw based on the electroencephalogram of the person when listening to the voice reading the sentence and the start point of each word included in the sentence.

The subject electroencephalogram feature 121 is a component feature Ifa in the "electroencephalogram response to a word" of the learning subject generated based on the subject electroencephalogram 4 of the learning subject when listening to the voice uttering the sentence and the start point of each word included in the sentence.

The mood estimation program 82 further causes the estimation device 2 to execute an onset information acquisition step (S203) of acquiring the onset information 8 indicating the start point of each word included in the sentence that the target person is listening to as a voice. Then, the mood estimation program 82 causes the estimation device 2 to generate, as the target person electroencephalogram feature 221, the component feature Ifa in the "electroencephalogram response to a word" of the target person based on the target person electroencephalogram 6 and the "start point of each word included in the sentence that the target person is listening to as a voice" indicated by the onset information 8 in the electroencephalogram encoding step. The mood estimation program 82 causes the estimation device 2 to execute processing of estimating the target person mood score 223 by inputting, as the target person electroencephalogram feature 221, the component feature Ifa in the "electroencephalogram response to a word" of the target person to the estimation model 3 in the estimation step.

In this configuration, the electroencephalogram feature Fw is the component feature Ifa in the "electroencephalogram response to a word included in a sentence". The training data set 120 is formed by associating the subject mood score 123 with the component feature Ifa in the "electroencephalogram response to a word" of the learning subject. Then, the estimation model 3 is generated by performing machine learning using the training data set 120. Therefore, the estimation model 3 can estimate the mood score Sm indicating the level of person depressed mood from the component feature Ifa in the "electroencephalogram response to a word included in a sentence".

Further, the estimation device 2 executes the onset information acquisition step of acquiring the onset information 8. The estimation device 2 generates the component feature Ifa in the "electroencephalogram response to a word" of the target person as the target person electroencephalogram feature 221 based on the target person electroencephalogram 6 and the onset information 8. Then, the estimation device 2 estimates the target person mood score 223 by inputting the component feature Ifa in the "electroencephalogram response to a word" of the target person to the estimation model 3.

Therefore, the estimation device 2 can estimate the target person mood score 223 indicating the level of the depressed mood of the target person based on the electroencephalogram (the target person electroencephalogram 6) of the target person when listening to the voice uttering the sentence and the onset information 8 indicating the start point of each word included in the sentence.

In the mood estimation program 82, the estimation model 3 may further receive, as an input, the subjective score Ss indicating subjective evaluation felt by a person for a sentence after listening to a voice uttering the sentence, in addition to the electroencephalogram feature Fw, and estimate the mood score Sm based on the electroencephalogram feature Fw and the subjective score Ss that are input.

Each of the plurality of training data sets 120 is formed by associating the subject mood score 123 with the subject electroencephalogram feature 121 and the subject subjective score 122 that is the subjective score Ss indicating the subjective evaluation felt by the learning subject for a sentence after listening to the voice uttering the sentence. Performing the machine learning includes a training step of training the estimation model 3 so that the mood score Sm estimated by the estimation model 3 when the subject electroencephalogram feature 121 and the subject subjective score 122 are input matches the subject mood score 123 for each of the plurality of training data sets 120.

The mood estimation program 82 further causes the estimation device 2 to execute a target person subjective score acquisition step (S204) of acquiring the target person subjective score 9 that is the subjective score Ss indicating the subjective evaluation felt by the target person for a sentence after listening to the voice uttering the sentence. Then, the mood estimation program 82 causes the estimation device 2 to execute processing of estimating the target person mood score 223 by inputting the target person electroencephalogram feature 221 and the target person subjective score 9 to the estimation model 3 in the estimation step.

In this configuration, the training data set 120 is formed by associating the subject mood score 123 with the subject electroencephalogram feature 121 and the subject subjective score 122. In addition, performing the machine learning includes a training step of training the estimation model 3 so that the mood score Sm estimated by the estimation model 3 when the subject electroencephalogram feature 121 and the subject subjective score 122 are input matches the subject mood score 123 for each of the plurality of training data sets 120. Therefore, the estimation model 3 can estimate the mood score Sm indicating the level of person depressed mood based on the electroencephalogram feature Fw and the subjective score Ss indicating the subjective evaluation felt by a person for the sentence listened as a voice. The present inventors verified the estimation accuracy for the estimation model 3 generated by performing machine learning using the plurality of training data sets 120 formed by associating the subject mood score 123 with the subject electroencephalogram feature 121 and the subject subjective score 122, respectively, and obtained the following verification results. That is, it was confirmed that the AUC of the estimation model 3 was "0.83". In addition, the estimation model 3 identified 78% of people with high level of depressive mood as having high level of depressive mood.

Further, the estimation device 2 executes a target person subjective score acquisition step of acquiring the target person subjective score 9. The estimation device 2 estimates the target person mood score 223 by inputting the target person electroencephalogram 6 and the target person subjective score 9 to the estimation model 3.

Therefore, the estimation device 2 can estimate the target person mood score 223 indicating the level of the depressed mood of the level of with high accuracy based on the electroencephalogram (the target person electroencephalogram 6) of the target person when listening to the voice uttering a sentence and the target person subjective score 9 indicating the subjective evaluation felt by the target person for the sentence.

### §4 Modified example

Although the embodiments have been described in detail above, the above description is merely an example of the present invention in all respects. It goes without saying that various modifications or variations can be made without departing from the scope of the present invention. For example, the following modifications are possible. In the following description, the same reference numerals are used for the same structural elements as those of the above embodiment, and the description of the same points as those of the above embodiment is appropriately omitted. The following modifications can be appropriately combined.

<4.1>
In the above embodiment, an example has been described in which the component feature Ifa of a "electroencephalogram response to a word" of a person is used as the electroencephalogram feature Fw generated from the electroencephalogram (the electroencephalogram response) of the person when listening to the voice uttering a sentence. However, in the present invention, it is not essential that the electroencephalogram feature Fw be the component feature Ifa of the "electroencephalogram response to a word". In the present invention, the electroencephalogram feature Fw may be the component feature Ifa of at least one of "electroencephalogram response to a word" and "electroencephalogram response following a voice envelope".

That is, the mood estimation program 82 may cause the estimation device 2 to generate, in the electroencephalogram encoding step (step S205), the component feature Ifa in the "electroencephalogram response to a word" instead of the component feature Ifa in the "electroencephalogram response to a word" or together with the component feature Ifa in the "electroencephalogram response to a word", the component feature Ifa in the "electroencephalogram response following a voice envelope". For example, the component feature Ifa in the "electroencephalogram response following the voice" of the person may be generated as the electroencephalogram feature Fw from the electroencephalogram of the person when listening to the voice uttering the sentence. Specifically, the estimation device 2 (in particular, the encoding unit 215) may generate, as the electroencephalogram feature Fw, the component feature Ifa in the "electroencephalogram response following the voice" of the person from the electroencephalogram of the person while listening to the voice reading the sentence and the voice envelope of the voice.

Correspondingly, the subject electroencephalogram feature 121 is regarded as the component feature Ifa in the "electroencephalogram response following the voice envelope" of the learning subject generated from the electroencephalogram of the learning subject when listening to the voice uttering the sentence and the voice envelope of the voice. In this case, the mood estimation program 82 further causes the estimation device 2 to execute an envelope information acquisition step of acquiring envelope information indicating the voice envelope of the voice the target person is listening to. The envelope information acquisition step may be performed instead of the onset information acquisition step (S203) or together with the onset information acquisition step (S203).

The mood estimation program 82 causes the estimation device 2 to generate, as the target person electroencephalogram feature 221, the component feature Ifa in the "electroencephalogram response following the voice envelope" of the target person based on the target person electroencephalogram 6 and the "voice envelope of the voice the target person is listening to" indicated by the above-described envelope information in the electroencephalogram encoding step (step S205). Then, the mood estimation program 82 causes the estimation device 2 to execute processing of estimating the target person mood score 223 by inputting, as the target person electroencephalogram feature 221, the component feature Ifa in the "electroencephalogram response following the voice envelope" of the target person to the estimation model 3 in the estimation step (step S206).

In this configuration, the electroencephalogram feature Fw is the component feature Ifa in the "electroencephalogram response following the voice envelope of the listening voice". The training data set 120 is formed by associating the subject mood score 123 with the component feature Ifa in the "electroencephalogram response following the voice envelope" of the learning subject. Then, the estimation model 3 is generated by performing machine learning using the training data set 120. Therefore, the estimation model 3 can estimate the mood score Sm indicating the level of depressed mood of a person from the component feature Ifa in the "electroencephalogram response following the voice envelope" of the person.

Further, the estimation device 2 executes the envelope information acquisition step of acquiring envelope information. The estimation device 2 generates the component feature Ifa in the "electroencephalogram response following the voice envelope" of the target person as the target person electroencephalogram feature 221 based on the target person electroencephalogram 6 and the envelope information. Then, the estimation device 2 estimates the target person mood score 223 by inputting the component feature Ifa in the "electroencephalogram response following the voice envelope" of the target person to the estimation model 3.

Therefore, the estimation device 2 can estimate the target person mood score 223 indicating the level of the depressed mood of the target person based on the electroencephalogram of the target person (the target person electroencephalogram 6) when listening to the voice uttering the sentence and the envelope information indicating the voice envelope of the voice.

<4.2>
In the above embodiment, an example has been described in which a sentence (for example, news) that a person is listening to as a voice is classified into any of the three categories of "Ng", "Nt", and "Ps". However, in the present disclosure, it is not essential that a sentence that a person is listening to as a voice is classified into any of the three categories of "Ng", "Nt", and "Ps". In the present disclosure a sentence that a person is listening to as a voice may be classified into any of at least three categories of "Ng", "Nt", and "Ps", and may be classified into any of four or more categories.

### Reference Signs List

2 Estimation device (computer)
3 Estimation model
6 Target person electroencephalogram
7 Classification information
8 Onset information
9 Target person subjective score
82 Mood estimation program
121 Subject electroencephalogram feature
121(1) Subject first electroencephalogram feature
121(2) Subject second electroencephalogram feature
121(3) Subject third electroencephalogram feature
122 Subject subjective score
123 Subject mood score
221 Target person electroencephalogram feature
223 Target person mood score
Fw electroencephalogram feature
Sm Mood score
Ss Subjective score
S201 Target person electroencephalogram acquisition step
S202 Classification information acquisition step
S203 Onset information acquisition step
S204 Target person subjective score acquisition step
S205 Electroencephalogram encoding step
S206 Estimation step
S207 Output step

## Claims

1. A mood estimation computer program comprising instructions which, when the program is executed by a computer, causing a computer to perform :
a target person electroencephalogram acquisition step of acquiring a target person electroencephalogram which is an electroencephalogram of a target person when listening to a voice uttering a sentence;
an electroencephalogram encoding step of generating an electroencephalogram feature from an electroencephalogram of a person when listening to a voice uttering a sentence, the electroencephalogram encoding step generating a target person electroencephalogram feature as the electroencephalogram feature from the target person electroencephalogram; and
an estimation step of estimating a target person mood score which is a mood score indicating a level of depressed mood of the target person by inputting the target person electroencephalogram feature to an estimation model,
the estimation model receiving at least the electroencephalogram feature as an input and estimating a mood score indicating a level of depressed mood of the person,
the estimation model being generated by performing machine learning using a plurality of training data sets,
each of the plurality of training data sets being formed by
associating a subject mood score which is the mood score indicating a level of depressed mood of a learning subject with at least a subject electroencephalogram feature which is the electroencephalogram feature generated from an electroencephalogram of the learning subject when listening to a voice uttering a sentence,
performing the machine learning including a training step of training the estimation model so that the mood score estimated by the estimation model when the subject electroencephalogram feature is received as an input matches the subject mood score for each of the plurality of training data sets.

2. The mood estimation program according to claim 1, causing the computer to perform
in the electroencephalogram encoding step, generating an electroencephalogram feature corresponding to a category into which the sentence is classified, as the electroencephalogram feature, based on
an electroencephalogram of a person when listening to a voice uttering a sentence classified into any of at least three categories of negative, neutral, and positive, and
information indicating into which of the at least three categories the sentence is classified,
the estimation model estimating the mood score when an electroencephalogram feature corresponding to a category into which the sentence is classified is input as the electroencephalogram feature,
the subject electroencephalogram feature including
a subject first electroencephalogram feature which is an electroencephalogram feature corresponding to the category of negative generated from an average of a plurality of electroencephalograms each of which is an electroencephalogram of the learning subject when listening to a voice uttering a sentence classified into the category of negative,
a subject second electroencephalogram feature which is an electroencephalogram feature corresponding to the category of neutral generated from an average of a plurality of electroencephalograms each of which is an electroencephalogram of the learning subject when listening to a voice uttering a sentence classified into the category of neutral, and
a subject third electroencephalogram feature which is an electroencephalogram feature corresponding to the category of positive generated from an average of a plurality of electroencephalograms each of which is an electroencephalogram of the learning subject when listening to a voice uttering a sentence classified into the category of positive,
performing the machine learning including, for each of the plurality of training data sets,
a first training step of training the estimation model so that the mood score estimated by the estimation model when the subject first electroencephalogram feature is input as the electroencephalogram feature corresponding to the category of negative matches the subject mood score,
a second training step of training the estimation model so that the mood score estimated by the estimation model when the subject second electroencephalogram feature is input as the electroencephalogram feature corresponding to the category of neutral matches the subject mood score, and
a third training step of training the estimation model so that the mood score estimated by the estimation model when the subject third electroencephalogram feature is input as the electroencephalogram feature corresponding to the category of positive matches the subject mood score,
the program causing the computer to further perform a classification information acquisition step of acquiring classification information indicating into which of the at least three categories a sentence the target person is listening to as a voice is classified, and
in the estimation step, estimating the target person mood score by inputting to the estimation model, as an electroencephalogram feature corresponding to a category indicated by the classification information, the target person electroencephalogram feature corresponding to a category indicated by the classification information generated, in the electroencephalogram encoding step, based on
the target person electroencephalogram of the target person when listening to a voice uttering a sentence classified into a category indicated by the classification information and
the classification information.

3. The mood estimation program according to claim 1 or 2, causing the computer to perform in the electroencephalogram encoding step, generating, as the electroencephalogram feature, at least one of a peak latency and an average amplitude before and after a peak of a predetermined component in an electroencephalogram response to a word of a person based on
an electroencephalogram of the person when listening to a voice uttering a sentence and
a start point of each word included in the sentence that the person is listening to as a voice,
the subject electroencephalogram feature being at least one of a peak latency and an average amplitude before and after a peak of the predetermined component in an electroencephalogram response to the word of the learning subject generated based on
an electroencephalogram of the learning subject when listening to a voice uttering a sentence and
a start point of each word included in the sentence that the learning subject is listening to as a voice,
the program causing the computer to further perform an onset information acquisition step of acquiring onset information indicating a start point of each word included in a sentence that the target person is listening to as a voice, and
in the estimation step, estimating the target person mood score by inputting to the estimation model, as the target person electroencephalogram feature, at least one of a peak latency and an average amplitude before and after a peak of the predetermined component in an electroencephalogram response to the word of the target person generated, in the electroencephalogram encoding step, based on
the target person electroencephalogram and
a start point of each word included in a sentence that the target person is listening to as a voice indicated by the onset information.

4. The mood estimation program according to claim 1 or 2, causing the computer to perform in the electroencephalogram encoding step, generating, as the electroencephalogram feature, at least one of a peak latency and an average amplitude before and after a peak of a predetermined component in an electroencephalogram response following a voice envelope of a person based on
an electroencephalogram of the person when listening to a voice uttering a sentence and
the voice envelope of the voice that the person is listening to,
the subject electroencephalogram feature being at least one of a peak latency and an average amplitude before and after a peak of the predetermined component in an electroencephalogram response following the voice envelope of the learning subject generated based on
an electroencephalogram of the learning subject when listening to a voice uttering a sentence and
a voice envelope of the voice that the learning subject was listening to,
the program causing the computer to further perform an envelope information acquisition step of acquiring envelope information indicating a voice envelope of a voice the target person is listening to, and
in the estimation step, estimating the target person mood score by inputting to the estimation model, as the target person electroencephalogram feature, at least one of a peak latency and an average amplitude before and after a peak of the predetermined component in an electroencephalogram response following the voice envelope of the target person generated, in the electroencephalogram encoding step, based on
the target person electroencephalogram and
a voice envelope of a voice the target person is listening to indicated by the envelope information.

5. The mood estimation program according to claim 1 or 2, wherein
the estimation model further receives, as an input, a subjective score indicating subjective evaluation felt by a person for a sentence after listening to a voice uttering the sentence, in addition to the electroencephalogram feature, and estimates the mood score based on the electroencephalogram feature and the subjective score that are input,
each of the plurality of training data sets is formed by associating
the subject mood score with the subject electroencephalogram feature and
a subject subjective score which is the subjective score indicating subjective evaluation felt by the learning subject for the sentence after listening to a voice uttering the sentence,
performing the machine learning includes a training step of training the estimation model so that the mood score estimated by the estimation model when the subject electroencephalogram feature and the subject subjective score are input matches the subject mood score for each of the plurality of training data sets,
the program causing a computer to further perform a target person subjective score acquisition step of acquiring a target person subjective score which is the subjective score indicating subjective evaluation that the target person felt for the sentence after listening to a voice uttering the sentence, and
in the estimation step, estimating the target person mood score by inputting to the estimation model,
the target person electroencephalogram feature and
the target person subjective score.

6. The mood estimation program according to claim 1 or 2, causing the computer to further perform an output step of outputting, to the target person, information corresponding to the target person mood score estimated in the estimation step.

## Patentansprüche

1. Stimmungsschätzungscomputerprogramm, umfassend Befehle, die, wenn das Programm durch einen Computer ausgeführt wird, bewirken, dass der Computer Folgendes durchführt:
einen Zielperson-Elektroenzephalogramm-Erfassungsschritt des Erfassens eines Zielperson-Elektroenzephalogramms, das ein Elektroenzephalogramm einer Zielperson, während sie einer einen Satz aussprechenden Stimme zuhört, ist;
einen Elektroenzephalogramm-Kodierungsschritt des Erzeugens eines Elektroenzephalogramm-Merkmals aus einem Elektroenzephalogramm einer Person, während sie einer einen Satz aussprechenden Stimme zuhört, wobei der Elektroenzephalogramm-Kodierungsschritt als das Elektroenzephalogramm-Merkmal ein Zielperson-Elektroenzephalogramm-Merkmal aus dem Zielperson-Elektroenzephalogramm erzeugt; und
einen Schätzungsschritt des Schätzens eines Zielperson-Stimmungswerts, der ein Stimmungswert ist, welcher ein Ausmaß einer deprimierten Stimmung der Zielperson ist, durch Eingeben des Zielperson-Elektroenzephalogramm-Merkmals in ein Schätzungsmodell,
wobei das Schätzungsmodell zumindest das Elektroenzephalogramm-Merkmal als eine Eingabe empfängt und einen Stimmungswert schätzt, der ein Ausmaß einer deprimierten Stimmung der Person angibt,
wobei das Schätzungsmodell durch Durchführen von maschinellem Lernen unter Verwendung einer Vielzahl von Trainingsdatensätzen erzeugt wird,
wobei jeder aus der Vielzahl von Trainingsdatensätzen durch Folgendes ausgebildet wird:
Zuordnen eines Individuumsstimmungswerts, welcher der Stimmungswert ist, der ein Ausmaß einer deprimierten Stimmung eines lernenden Individuums angibt, zu zumindest einem Individuum-Elektroenzephalogramm-Merkmal, welches das Elektroenzephalogramm-Merkmal ist, das aus einem Elektroenzephalogramm des lernenden Individuums, während es einer einen Satz aussprechenden Stimme zuhört, erzeugt wurde,
Durchführen des maschinellen Lernens, umfassend einen Trainingsschritt des Trainierens des Schätzungsmodells, derart, dass der durch das Schätzungsmodell geschätzte Stimmungswert, wenn das Individuum-Elektroenzephalogramm-Merkmal als eine Eingabe empfangen wird, mit dem Individuumsstimmungswert für jeden aus der Vielzahl von Trainingsdatensätzen übereinstimmt.

2. Stimmungsschätzungsprogramm nach Anspruch 1, welches bewirkt, dass der Computer Folgendes durchführt:
im Elektroenzephalogramm-Kodierungsschritt Erzeugen eines Elektroenzephalogramm-Merkmals, das einer Kategorie entspricht, in welche der Satz klassifiziert ist, als das Elektroenzephalogramm-Merkmal, basierend auf
einem Elektroenzephalogramm einer Person, während sie einer Stimme zuhört, die einen Satz ausspricht, der in eine aus zumindest drei Kategorien, negativ, neutral und positiv, klassifiziert ist, und
Informationen, die angeben, in welche aus den zumindest drei Kategorien der Satz klassifiziert ist,
wobei das Schätzungsmodell den Stimmungswert schätzt, wenn ein Elektroenzephalogramm-Merkmal, das einer Kategorie, in welche der Satz klassifiziert ist, entspricht, als das Elektroenzephalogramm-Merkmal eingegeben wird,
wobei das Individuum-Elektroenzephalogramm-Merkmal Folgendes umfasst:
ein erstes Individuum-Elektroenzephalogramm-Merkmal, das ein der Kategorie negativ entsprechendes Elektroenzephalogramm-Merkmal ist, welches aus einem Durchschnitt einer Vielzahl von Elektroenzephalogrammen erzeugt wurde, die jeweils ein Elektroenzephalogramm des lernenden Individuums, während es einer Stimme zuhört, die einen Satz ausspricht, der in die Kategorie negativ klassifiziert ist, sind,
ein zweites Individuum-Elektroenzephalogramm-Merkmal, das ein der Kategorie neutral entsprechendes Elektroenzephalogramm-Merkmal ist, welches aus einem Durchschnitt einer Vielzahl von Elektroenzephalogrammen erzeugt wurde, die jeweils ein Elektroenzephalogramm des lernenden Individuums, während es einer Stimme zuhört, die einen Satz ausspricht, der in die Kategorie neutral klassifiziert ist, sind,
ein drittes Individuum-Elektroenzephalogramm-Merkmal, das ein der Kategorie positiv entsprechendes Elektroenzephalogramm-Merkmal ist, welches aus einem Durchschnitt einer Vielzahl von Elektroenzephalogrammen erzeugt wurde, die jeweils ein Elektroenzephalogramm des lernenden Individuums, während es einer Stimme zuhört, die einen Satz ausspricht, der in die Kategorie positiv klassifiziert ist, sind,
Durchführen des maschinellen Lernens, umfassend, für jeden aus der Vielzahl von Trainingsdatensätzen:
einen ersten Trainingsschritt des Trainierens des Schätzungsmodells derart, dass der durch das Schätzungsmodell geschätzte Stimmungswert, wenn das erste Individuum-Elektroenzephalogramm-Merkmal als das Elektroenzephalogramm-Merkmal, das der Kategorie negativ entspricht, eingegeben wird, mit dem Individuum-Stimmungswert übereinstimmt,
einen zweiten Trainingsschritt des Trainierens des Schätzungsmodells derart, dass der durch das Schätzungsmodell geschätzte Stimmungswert, wenn das zweite Individuum-Elektroenzephalogramm-Merkmal als das Elektroenzephalogramm-Merkmal, das der Kategorie neutral entspricht, eingegeben wird, mit dem Individuum-Stimmungswert übereinstimmt,
einen dritten Trainingsschritt des Trainierens des Schätzungsmodells derart, dass der durch das Schätzungsmodell geschätzte Stimmungswert, wenn das dritte Individuum-Elektroenzephalogramm-Merkmal als das Elektroenzephalogramm-Merkmal, das der Kategorie positiv entspricht, eingegeben wird, mit dem Individuum-Stimmungswert übereinstimmt,
wobei das Programm bewirkt, dass der Computer ferner einen Klassifizierungsinformationserfassungsschritt des Erfassens von Klassifizierungsinformationen durchführt, welche angeben, in welche aus den zumindest drei Kategorien ein Satz, dem die Zielperson als Stimme zuhört, klassifiziert ist, und
im Schätzungsschritt Schätzen des Zielperson-Stimmungswerts durch Eingeben in das Schätzungsmodell des Zielperson-Elektroenzephalogramm-Merkmals, das einer Kategorie entspricht, die durch die Klassifizierungsinformationen, die im Elektroenzephalogramm-Kodierungsschritt erzeugt wurden, angegeben wird, als ein Elektroenzephalogramm-Merkmal, das einer Kategorie entspricht, die durch die Klassifizierungsinformationen angegeben werden, basierend auf
dem Zielperson-Elektroenzephalogramm der Zielperson, während sie einer Stimme zuhört, die einen Satz ausspricht, welcher in eine durch die Klassifizierungsinformationen angezeigte Kategorie klassifiziert ist, und
den Klassifizierungsinformationen.

3. Stimmungsschätzungsprogramm nach Anspruch 1 oder 2, welches bewirkt, dass der Computer im Elektroenzephalogramm-Kodierungsschritt als das Elektroenzephalogramm-Merkmal zumindest eines aus einer Peak-Latenz und einer durchschnittlichen Amplitude vor und nach einem Peak einer vorbestimmten Komponente in einer Elektroenzephalogramm-Reaktion auf ein Wort einer Person basierend auf Folgendem erzeugt
einem Elektroenzephalogramm der Person, während sie einer einen Satz aussprechenden Stimme zuhört, und
einem Startpunkt jedes Worts, das in dem Satz enthalten ist, welchem die Person als Stimme zuhört,
wobei das Individuum-Elektroenzephalogramm-Merkmal zumindest eines aus einer Peak-Latenz und einer durchschnittlichen Amplitude vor und nach einem Peak der vorbestimmten Komponente in einer Elektroenzephalogramm-Reaktion auf das Wort des lernenden Individuums ist, die erzeugt wird basierend auf
einem Elektroenzephalogramm des lernenden Individuums, während es einer einen Satz aussprechenden Stimme zuhört, und
einem Startpunkt jedes Worts, das in dem Satz enthalten ist, welchem das lernende Individuum als eine Stimme zuhört,
wobei das Programm bewirkt, dass der Computer ferner einen Einsatzinformationserfassungsschritt des Erfassens von Einsatzinformationen ausführt, welche einen Startpunkt jedes Worts, das in einem Satz enthalten ist, welchem die Zielperson als Stimme zuhört, angeben, und
im Schätzungsschritt das Schätzen des Zielperson-Stimmungswerts durch Eingeben von zumindest einem aus einer Peak-Latenz und einer durchschnittlichen Amplitude vor und nach einem Peak der vorbestimmten Komponente in einer Elektroenzephalogramm-Reaktion auf das Wort der Zielperson, erzeugt im Elektroenzephalogramm-Kodierungsschritt, als das Zielperson-Elektroenzephalogramm-Merkmal in das Schätzungsmodell, auf Folgendem basierend:
dem Zielperson-Elektroenzephalogramm-Merkmal und
einem durch die Einsatzinformationen angegebenen Startpunkt jedes Worts, das in dem Satz enthalten ist, welchem die Zielperson als eine Stimme zuhört.

4. Stimmungsschätzungsprogramm nach Anspruch 1 oder 2, welches bewirkt, dass der Computer im Elektroenzephalogramm-Kodierungsschritt als das Elektroenzephalogramm-Merkmal zumindest eines aus einer Peak-Latenz und einer durchschnittlichen Amplitude vor und nach einem Peak einer vorbestimmten Komponente in einer Elektroenzephalogramm-Reaktion im Anschluss an eine Stimmhüllkurve einer Person basierend auf Folgendem erzeugt
einem Elektroenzephalogramm der Person, während sie einer einen Satz aussprechenden Stimme zuhört, und
der Stimmhüllkurve der Stimme, welcher die Person zuhört,
wobei das Individuum-Elektroenzephalogramm-Merkmal zumindest eines aus einer Peak-Latenz und einer durchschnittlichen Amplitude vor und nach einem Peak der vorbestimmten Komponente in einer Elektroenzephalogramm-Reaktion im Anschluss an eine Stimmhüllkurve des lernenden Individuums ist, basierend auf
einem Elektroenzephalogramm des lernenden Individuums, während es einer einen Satz aussprechenden Stimme zuhört, und
einer Stimmhüllkurve der Stimme, welcher das lernende Individuum zugehört hat,
wobei das Programm bewirkt, dass der Computer ferner einen Hüllkurveninformationserfassungsschritt des Erfassens von Hüllkurveninformationen, welche eine Stimmhüllkurve einer Stimme, welcher die Zielperson zuhört, angeben, durchführt, und
wobei im Schätzungsschritt das Schätzen des Zielperson-Stimmungswerts durch Eingeben in das Schätzungsmodell von zumindest einem aus einer Peak-Latenz und einer durchschnittlichen Amplitude vor und nach einem Peak der vorbestimmten Komponente in einer Elektroenzephalogramm-Reaktion im Anschluss an die Stimmhüllkurve der Zielperson, erzeugt im Elektroenzephalogramm-Kodierungsschritt, als das Zielperson-Elektroenzephalogramm-Merkmal auf Folgendem basiert
dem Zielperson-Elektroenzephalogramm und
einer durch die Hüllkurveninformationen angegebenen Stimmhüllkurve der Stimme, welcher die Zielperson zuhört.

5. Stimmungsschätzungsprogramm nach Anspruch 1 oder 2, wobei das Schätzungsmodell zusätzlich zu dem Elektroenzephalogramm-Merkmal ferner als eine Eingabe einen subjektiven Wert empfängt, der eine subjektive Bewertung angibt, welche von einer Person in Bezug auf einen Satz empfunden wird, nachdem sie einer den Satz aussprechenden Stimme zugehört hat, und den Stimmungswert basierend auf das Elektroenzephalogramm-Merkmal und dem subjektiven Wert, die eingegeben werden, schätzt,
wobei jeder aus der Vielzahl von Trainingsdatensätzen durch Zuordnen von Folgendem ausgebildet wird
dem Individuum-Stimmungswert zu dem Individuum-Elektroenzephalogramm-Merkmal und
einem subjektiven Individuumswert, welcher der subjektive Wert ist, der eine subjektive Bewertung angibt, welche von dem lernenden Individuum in Bezug auf den Satz empfunden wird, nachdem es einer den Satz aussprechenden Stimme zugehört hat,
wobei das Durchführen des maschinellen Lernens einen Trainingsschritt des Trainierens des Schätzungsmodells derart umfasst, dass der Stimmungswert, der von dem Schätzungsmodell geschätzt wird, wenn das Individuum-Elektroenzephalogramm-Merkmal und der subjektive Individuumswert eingegeben werden, mit dem Individuum-Stimmungswert für jeden aus der Vielzahl von Trainingsdatensätzen übereinstimmt,
wobei das Programm bewirkt, dass ein Computer ferner einen Subjektiver-Zielperson-Stimmungswert-Erfassungsschritt des Erfassens eines subjektiven Werts einer Zielperson umfasst, welcher der subjektive Wert ist, der die subjektive Bewertung angibt, welche von der Zielperson in Bezug auf den Satz empfunden wurde, nachdem sie einer den Satz aussprechenden Stimme zugehört hat, und
im Schätzungsschritt das Schätzen des Zielperson-Stimmungswerts durch Eingeben in das Schätzungsmodell
des Zielperson-Elektroenzephalogramm-Merkmals und
des subjektiven Zielperson-Werts.

6. Stimmungsschätzungsprogramm nach Anspruch 1 oder 2, welches bewirkt, dass der Computer ferner einen Ausgabeschritt des Ausgebens von Informationen, welche dem im Schätzungsschritt geschätzten Zielperson-Stimmungswert entsprechen, für die Zielperson durchführt.

## Revendications

1. Programme informatique d'estimation d'humeur comprenant des instructions qui, lorsque le programme est exécuté par un ordinateur, amènent un ordinateur à réaliser :
une étape d'acquisition d'électroencéphalogramme de personne cible consistant à acquérir un électroencéphalogramme de personne cible qui est un électroencéphalogramme d'une personne cible lors de l'écoute d'une voix prononçant une phrase ;
une étape de codage d'électroencéphalogramme consistant à générer une caractéristique d'électroencéphalogramme à partir d'un électroencéphalogramme d'une personne lors de l'écoute d'une voix prononçant une phrase, l'étape de codage d'électroencéphalogramme générant une caractéristique d'électroencéphalogramme de personne cible en tant que caractéristique d'électroencéphalogramme à partir de l'électroencéphalogramme de personne cible ; et
une étape d'estimation consistant à estimer un score d'humeur de la personne cible qui est un score d'humeur indiquant un niveau d'humeur déprimée de la personne cible en entrant la caractéristique d'électroencéphalogramme de la personne cible dans un modèle d'estimation,
le modèle d'estimation recevant au moins la caractéristique d'électroencéphalogramme en tant qu'entrée et estimant un score d'humeur indiquant un niveau d'humeur déprimée de la personne,
le modèle d'estimation étant généré en réalisant un apprentissage automatique en utilisant une pluralité d'ensembles de données d'entraînement,
chacun de la pluralité d'ensembles de données d'entraînement étant formé par
l'association d'un score d'humeur de sujet qui est le score d'humeur indiquant un niveau d'humeur déprimée d'un sujet d'apprentissage à au moins une caractéristique d'électroencéphalogramme de sujet qui est la caractéristique d'électroencéphalogramme générée à partir d'un électroencéphalogramme du sujet d'apprentissage lors de l'écoute d'une voix prononçant une phrase,
la réalisation de l'apprentissage automatique comprenant une étape d'entraînement consistant à entraîner le modèle d'estimation de sorte que le score d'humeur estimé par le modèle d'estimation lorsque la caractéristique d'électroencéphalogramme du sujet est reçue en tant qu'entrée corresponde au score d'humeur de sujet pour chacun de la pluralité d'ensembles de données d'entraînement.

2. Programme d'estimation d'humeur selon la revendication 1, amenant l'ordinateur à réaliser dans l'étape de codage d'électroencéphalogramme, la génération d'une caractéristique d'électroencéphalogramme correspondant à une catégorie dans laquelle la phrase est classée, en tant que caractéristique d'électroencéphalogramme, sur la base d'un
électroencéphalogramme d'une personne lorsqu'elle écoute une voix prononcer une phrase classée dans l'une quelconque d'au moins trois catégories suivantes : négative, neutre et positive, et
d'informations indiquant dans laquelle des au moins trois catégories la phrase est classée,
le modèle d'estimation estimant le score d'humeur lorsqu'une caractéristique d'électroencéphalogramme correspondant à une catégorie dans laquelle la phrase est classée est entrée en tant que caractéristique d'électroencéphalogramme,
la caractéristique d'électroencéphalogramme du sujet comprenant
une première caractéristique d'électroencéphalogramme de sujet qui est une caractéristique d'électroencéphalogramme correspondant à la catégorie négative générée à partir d'une moyenne d'une pluralité d'électroencéphalogrammes dont chacun est un électroencéphalogramme du sujet d'apprentissage lors de l'écoute d'une voix prononçant une phrase classée dans la catégorie négative,
une deuxième caractéristique d'électroencéphalogramme de sujet qui est une caractéristique d'électroencéphalogramme correspondant à la catégorie neutre générée à partir d'une moyenne d'une pluralité d'électroencéphalogrammes dont chacun est un électroencéphalogramme du sujet d'apprentissage lors de l'écoute d'une voix prononçant une phrase classée dans la catégorie neutre, et
une troisième caractéristique d'électroencéphalogramme de sujet qui est une caractéristique d'électroencéphalogramme correspondant à la catégorie positive générée à partir d'une moyenne d'une pluralité d'électroencéphalogrammes dont chacun est un électroencéphalogramme du sujet d'apprentissage lors de l'écoute d'une voix prononçant une phrase classée dans la catégorie positive,
la réalisation de l'apprentissage automatique comprenant, pour chacun de la pluralité d'ensembles de données d'entraînement,
une première étape d'entraînement consistant à entraîner le modèle d'estimation de sorte que le score d'humeur estimé par le modèle d'estimation lorsque la première caractéristique d'électroencéphalogramme du sujet est entrée en tant que caractéristique d'électroencéphalogramme correspondant à la catégorie négative corresponde au score d'humeur du sujet,
une deuxième étape d'entraînement consistant à entraîner le modèle d'estimation de sorte que le score d'humeur estimé par le modèle d'estimation lorsque la deuxième caractéristique d'électroencéphalogramme du sujet est entrée en tant que caractéristique d'électroencéphalogramme correspondant à la catégorie neutre corresponde au score d'humeur du sujet, et
une troisième étape d'entraînement consistant à entraîner le modèle d'estimation de sorte que le score d'humeur estimé par le modèle d'estimation lorsque la troisième caractéristique d'électroencéphalogramme du sujet est entrée en tant que caractéristique d'électroencéphalogramme correspondant à la catégorie positive corresponde au score d'humeur du sujet,
le programme amenant l'ordinateur à réaliser en outre une étape d'acquisition d'informations de classification consistant à acquérir des informations de classification indiquant dans laquelle des au moins trois catégories une phrase que la personne cible écoute en tant que voix est classée, et
dans l'étape d'estimation, l'estimation du score d'humeur de la personne cible en entrant dans le modèle d'estimation, en tant que caractéristique d'électroencéphalogramme correspondant à une catégorie indiquée par les informations de classification, la caractéristique d'électroencéphalogramme de la personne cible correspondant à une catégorie indiquée par les informations de classification générées, dans l'étape de codage de l'électroencéphalogramme, sur la base de
l'électroencéphalogramme de la personne cible lors de l'écoute d'une voix prononçant une phrase classée dans une catégorie indiquée par les informations de classification et
Des informations de classification.

3. Programme d'estimation d'humeur selon la revendication 1 ou 2, amenant l'ordinateur à réaliser l'étape de codage d'électroencéphalogramme, en générant, en tant que caractéristique d'électroencéphalogramme, une latence de pic et/ou une amplitude moyenne avant et après un pic d'une composante prédéterminée dans une réponse d'électroencéphalogramme à un mot d'une personne sur la base d'
un électroencéphalogramme de la personne lors de l'écoute d'une voix prononçant une phrase et
d'un point de départ de chaque mot inclus dans la phrase que la personne écoute en tant que voix,
la caractéristique d'électroencéphalogramme du sujet étant une latence de pic et/ou une amplitude moyenne avant et après un pic de la composante prédéterminée dans une réponse d'électroencéphalogramme au mot du sujet d'apprentissage généré sur la base d'
un électroencéphalogramme du sujet d'apprentissage lors de l'écoute d'une voix prononçant une phrase et
d'un point de départ de chaque mot compris dans la phrase que le sujet d'apprentissage écoute en tant que voix,
le programme amenant l'ordinateur à réaliser en outre une étape d'acquisition d'informations d'apparition consistant à acquérir des informations d'apparition indiquant un point de départ de chaque mot compris dans une phrase que la personne cible écoute en tant que voix, et
dans l'étape d'estimation, à estimer le score d'humeur de la personne cible en entrant dans le modèle d'estimation, en tant que caractéristique d'électroencéphalogramme de la personne cible, une latence de pic et/ou une amplitude moyenne avant et après un pic de la composante prédéterminée dans une réponse d'électroencéphalogramme au mot de la personne cible généré, dans l'étape de codage d'électroencéphalogramme, sur la base de
l'électroencéphalogramme de la personne cible et
d'un point de départ de chaque mot compris dans une phrase que la personne cible écoute en tant que voix indiquée par les informations d'apparition.

4. Programme d'estimation d'humeur selon la revendication 1 ou 2, amenant l'ordinateur à réaliser l'étape de codage d'électroencéphalogramme, en générant, en tant que caractéristique d'électroencéphalogramme, une latence de pic et/ou une amplitude moyenne avant et après un pic d'une composante prédéterminée dans une réponse d'électroencéphalogramme suivant une enveloppe vocale d'une personne sur la base d'
un électroencéphalogramme de la personne lors de l'écoute d'une voix prononçant une phrase et
De l'enveloppe vocale de la voix que la personne écoute,
la caractéristique d'électroencéphalogramme du sujet étant une latence de pic et/ou une amplitude moyenne avant et après un pic de la composante prédéterminée dans une réponse d'électroencéphalogramme suivant l'enveloppe vocale du sujet d'apprentissage générée sur la base d'
un électroencéphalogramme du sujet d'apprentissage lors de l'écoute d'une voix prononçant une phrase et
d'une enveloppe vocale de la voix que le sujet d'apprentissage écoutait,
le programme amenant l'ordinateur à réaliser en outre une étape d'acquisition d'informations d'enveloppe consistant à acquérir des informations d'enveloppe indiquant une enveloppe vocale d'une voix que la personne cible écoute, et
dans l'étape d'estimation, à estimer le score d'humeur de la personne cible en entrant dans le modèle d'estimation, en tant que caractéristique d'électroencéphalogramme de la personne cible, une latence de pic et/ou une amplitude moyenne avant et après un pic de la composante prédéterminée dans une réponse d'électroencéphalogramme suivant l'enveloppe vocale de la personne cible générée, dans l'étape de codage d'électroencéphalogramme, sur la base de
l'électroencéphalogramme de la personne cible et
d'une enveloppe vocale d'une voix que la personne cible écoute indiquée par les informations de l'enveloppe.

5. Procédé d'estimation d'humeur selon la revendication 1 ou 2, dans lequel :
le modèle d'estimation reçoit en outre, en tant qu'entrée, un score subjectif indiquant une évaluation subjective ressentie par une personne pour une phrase après avoir écouté une voix prononcer la phrase, en plus de la caractéristique d'électroencéphalogramme, et estime le score d'humeur sur la base de la caractéristique d'électroencéphalogramme et du score subjectif qui sont entrés,
chacun de la pluralité d'ensembles de données d'entraînement est formé en associant
le score d'humeur du sujet à la caractéristique d'électroencéphalogramme du sujet et
un score subjectif du sujet qui est le score subjectif indiquant l'évaluation subjective ressentie par le sujet d'apprentissage pour la phrase après avoir écouté une voix prononcer la phrase,
la réalisation de l'apprentissage automatique comprend une étape d'entraînement consistant à entraîner le modèle d'estimation de sorte que le score d'humeur estimé par le modèle d'estimation lorsque la caractéristique d'électroencéphalogramme de sujet et le score subjectif du sujet sont entrés corresponde au score d'humeur du sujet pour chacun de la pluralité d'ensembles de données d'entraînement,
le programme amenant un ordinateur à réaliser en outre une étape d'acquisition de score subjectif d'une personne cible consistant à acquérir un score subjectif d'une personne cible qui est le score subjectif indiquant l'évaluation subjective que la personne cible a ressentie pour la phrase après avoir écouté une voix prononcer la phrase, et
dans l'étape d'estimation, à estimer le score d'humeur de la personne cible en entrant dans le modèle d'estimation
la caractéristique d'électroencéphalogramme de la personne cible et
le score subjectif de la personne cible.

6. Programme d'estimation d'humeur selon la revendication 1 ou 2, amenant l'ordinateur à réaliser en outre une étape de sortie consistant à délivrer, à la personne cible, des informations correspondant au score d'humeur de la personne cible estimé dans l'étape d'estimation.
